# EUROPEAN PATENT SPECIFICATION

(11) **EP 1 751 550 B1**
(45) Date of publication and mention of the grant of the patent: **13.05.2020**
(21) Application number: 05736052.1
(22) Date of filing: 14.04.2005
(51) Int. Cl.: G01N 33/48

(54) **LIVER DISEASE DIAGNOSIS SYSTEM, METHOD AND GRAPHICAL USER INTERFACE**
SYSTEM, VERFAHREN UND GRAPHISCHE BENUTZEROBERFLÄCHE ZUR DIAGNOSE VON LEBERKRANKHEITEN
SYSTEME, PROCEDE ET INTERFACE GRAPHIQUE PERMETTANT DE DIAGNOSTIQUER UNE MALADIE DU FOIE

(30) Priority: 14.04.2004 US 561921 P
(43) Date of publication of application: 14.02.2007
(73) Proprietor: Edda Technology, Inc., Princeton, NJ 08540 (US)
(72) Inventor: WEI, Guo-Qing, Plainsboro, NJ 08536 (US); QIAN, Jian-Zhong, Princeton Junction, NJ 08550 (US); FAN, Li, Belle Meade, NJ 08502 (US); LIANG, Cheng-Chung, West Windsor, NJ 08550 (US)
(74) Representative: Müller-Boré & Partner Patentanwälte PartG mbB
(86) International application number: PCT/US2005/012733
(87) International publication number: WO 2005/106474

(56) References cited:
- EP-A1- 1 127 547
- WO-A1-03/101303
- WO-A2-01/22363
- US-A- 5 891 454
- US-A1- 2003 013 959
- US-A1- 2003 095 697
- US-B2- 6 678 399
- US-B2- 6 944 330

## Description

The present invention claims priority of provisional patent application No. 60/561,921 filed Apr. 14, 2004.

### BACKGROUND OF THE INVENTION

### 1. Field of the Invention

The present invention relates generally to systems and methods for medical diagnosis. Specifically, the present invention relates to systems and methods for computer assisted medical diagnosis.

### 2. Description of Related Art

WO 03/101303 A1 discloses an adjunctive ultrasound mammography system and associated methods, comprising a scanning apparatus facilitating standardized, repeatable breast ultrasound scans, and an adjunctive ultrasound display apparatus configured for fast, intuitive viewing of adjunctive ultrasound data concurrently with x-ray mammogram information. Thick-slice ultrasound images are displayed near x-ray mammogram images for assistance in interpreting the x-ray mammogram images. Concurrent acquisition and display of vibrational resonance image (VDI) data is disclosed. Computer-aided diagnosis (CAD) algorithms incorporating acoustically-based feature vectors are disclosed. Easy navigation among thick-slice image arrays, enlarged thick-slice images, and planar ultrasound views is disclosed. Thick-slice images inverted prior to display are disclosed. Algorithms for computing thick-slice images from volumetric scan data are disclosed including feature enhancement algorithms, solutions for border-straddling lesions, and neighborhood-dependent integration algorithms for feature emphasis. Overlaying of thick slice ultrasound images with x-ray mammogram images for facilitating comprehension of breast structures and detecting abnormalities is disclosed.

US 2003/0095697 A1 discloses a computer-aided diagnostic method and system that provide image annotation information that can include an assessment of the probability, likelihood or predictive value of detected or identified suspected abnormalities as an additional aid to the radiologist. More specifically, probability values, in numerical form and/or analog form, are added to the locational markers of the detected and suspected abnormalities. The task of a physician using such a system as disclosed is believed to be made easier by displaying markers representing different regions of interest.

Early detection of liver cancer has recently become possible due to rapid technical advancement in diagnostic imaging systems. Detection and diagnosis of liver cancer usually involves multiple image acquisitions in, frequently, multiple image modalities. For example, Computerized Tomography (CT) is the most popular modality for earlier liver cancer detection and diagnosis. When CT images are used, up to four phases of images may be acquired for diagnosis purposes. These four phases include plain CT images, arterial phase images, portal venous phase images, and delayed phase images. When CT images are not adequate to assist in reaching a diagnosis, images in other image modalities may also be used. Examples of other modalities include images from Magnetic Resonance Imaging (MRI) or Positron Emission Tomography (PET). When a large amount of data becomes available, there is a need for means to make effective use of such data and to assist physicians or other medical personnel to improve throughput.

### SUMMARY

The present invention provides a system for liver disease diagnosis as set out in Claim 1.

The present invention also provides a method as set out in Claim 50.

### BRIEF DESCRIPTION OF THE DRAWINGS

The invention claimed and/or described herein is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
Fig. 1 depicts an exemplary construct of a system for computer assisted liver disease diagnosis, according to an embodiment of the present invention;
Fig. 2 shows an exemplary layout of a data manipulation page, according to an embodiment of the present invention;
Fig. 3 shows an exemplary layout of a data manipulation control area, according to an embodiment of the present invention;
Fig. 4 shows a different exemplary layout of a data manipulation page, according to an embodiment of the present invention;
Fig. 5 shows a different exemplary layout of a data manipulation control area, according to an embodiment of the present invention;
Fig. 6 shows an exemplary layout of a liver disease diagnosis page, according to an embodiment of the present invention;
Fig. 7(a) illustrates an exemplary hierarchical representation of diagnostic information, according to an embodiment of the present invention;
Fig. 7(b) illustrates an exemplary tabular layout of a diagnostic information display interface, according to an embodiment of the present invention;
Fig. 7(c) illustrates an exemplary tabular layout of a diagnostic information summary interface, according to an embodiment of the present invention;
Fig. 8 shows an exemplary layout of a diagnosis panel, according to an embodiment of the present invention;
Fig. 9 shows an exemplary hierarchy of diagnostic information organized as a tree, according to an embodiment of the present invention;
Fig. 10 shows an exemplary diagnostic tree for different types of liver diseases, according to an embodiment of the present invention;
Fig. 11 shows an exemplary tabular display of diagnostic information with indication of match against a specific disease type, according to an embodiment of the present invention.
Fig. 12 shows an exemplary interface for applying an embedded data manipulation tool to modify diagnostic information, according to an embodiment of the present invention;
Fig. 13 shows an exemplary layout of a reporting page, according to an embodiment of the present invention; and
Fig. 14 shows an exemplary layout of a portion of a reporting page, according to an embodiment of the present invention.

### DETAILED DESCRIPTION

The present invention relates to a system and method for liver disease diagnosis. A system and graphical user interfaces are disclosed herein that facilitate coordinated retrieval of visual and non-visual data associated with a patient and a liver disease, manipulation of visual/non-visual data to extract diagnostic information, generation of a hierarchical representation for visual and non-visual diagnostic information, interactive exploration of the hierarchy of diagnostic information, and an interactive diagnosis process. Method and system for effective visualization of data in different dimensions are also disclosed.

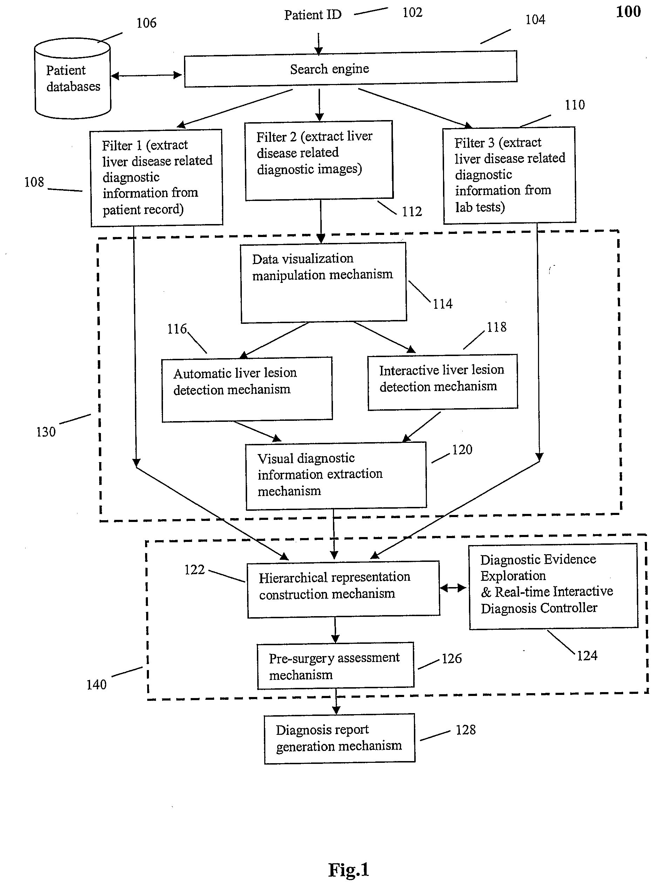

Fig. 1 depicts an exemplary construct of a system 100 for computer assisted liver disease diagnosis, according to an embodiment of the present invention. In this exemplary construct, the system 100 comprises a plurality of filters (a filter 1 108, a filter 2 112, and a filter 3 110), a visual data manipulation mechanism 130, a liver disease diagnosis mechanism 140, and a diagnosis report generation mechanism 128. The system 100 may further include a search engine 104 that retrieves information associated with a patient ID 102 from a patient database 106. The search engine 104 may access information stored in the patient database according to the patient ID 102 received. The patient database 106 may be a local data depository or a remote data depository. The patient database 106 may be a single database or multiple databases, which may be located at a single site or distributed at multiple locations across a network.

Information stored in the patient database 106 may include general patient information such as name, address, age, gender, or family history with regard to different diseases. The patient database 106 may also store information related to different medical tests and examinations. For example, blood test results measuring different organ functions may be stored in the patient database 106. Imageries acquired for medial examination purposes may also be stored in the patient database 106. For instance, visual images/volumes from MRI scans, CT, or PET may be stored in the patient database 106. In some embodiments, information stored in the patient database 106 may be indexed according to, for instance, patient ID, age, or an underlying disease suspected at the time the data is created. In some embodiments, cross or multiple indexing may also be made in the patient database 106 so that a user may query based on multiple conditions. For example, one may search with respect to a particular patient and a specific disease (e.g., liver disease).

In some embodiments, upon receiving the patient ID 102, the search engine 104 may retrieve all information associated with the patient ID. In other embodiments, the search engine 104 may be capable of selectively retrieving a portion of the information associated with the given patient ID 102 according to some criterion. For example, the search engine 104 may also be used as a filter to selectively retrieve data from the patient database 106. In the exemplary system 100, the three filters (108, 112, and 110) are provided that may function as a filter in order to select data that is appropriate and relevant to liver disease diagnosis.

In the exemplary system 100, the filter 1 108 is provided to filter a patient record to extract information related to liver disease and a diagnosis thereof. For example, information in a patient record relating to liver disease (e.g., age, symptoms, medication history, a history of hepatic diseases, an alcohol consumption level, a cancer history, and/or a family history of liver problems) may be identified as relevant to liver disease diagnosis and may be extracted. The filter 3 110 may be provided to filter various medical test results to extract information that is relevant to liver disease diagnosis. Such medical tests may include, for instance, a blood test for liver function (e.g., hematocrit, hemoglobin, platelet count, white blood cell count, carcinoembryonic antigen (CEA), or alpha fetoprotein (AFP)). Information filtered via the filter 108 and 110 may be non-visual information relevant to liver disease.

The filter 2 112 may be provided to filter visual data retrieved from the patient database 106 and retain those that are related to liver disease or relevant to a diagnosis for a liver disease. For example, X-ray images acquired to examine a patient's lung may not be relevant to diagnosis of a liver disease and may be filtered out by the filter 112. Visual diagnostic information for liver diseases may be imagery data (2D images or 3D volumes) in different modalities acquired via different imaging processes such as Ultrasound (US), CT, and MRI. In some embodiments, imagery data in a particular modality may be acquired at different times or phases. For example, CT images may include images from multiple phases, such as images from a plain CT phase, images from an arterial phase, images from a portal venous phase, or images from a delayed phase. Images of each phase may reveal different types of diagnostic information and different visualization techniques may be needed in order to effectively reveal such diagnostic information contained therein.

In some embodiments, visual information filtered through the filter 112 may be forwarded to the visual data manipulation mechanism 130, which may facilitate different data manipulation operations to be performed on the visual information. Such operations may include visualizations of the visual information and data processing. In the exemplary construct of the system 100, the visual data manipulation mechanism 130 comprises a data visualization/manipulation mechanism 114, an automatic liver lesion detection mechanism 116, an interactive liver lesion detection mechanism 118, and a visual diagnostic information extraction mechanism 120. The data visualization/manipulation mechanism 114 may be provided to facilitate different operations to be performed on imagery information. For example, the data visualization/manipulation mechanism 114 may render a graphical user interface (e.g., a visual data manipulation page) through which a user may control how visual data is to be visualized/manipulated, visualize data according to user's instructions, and effectuate data processing in accordance with user's interactions with the interface.

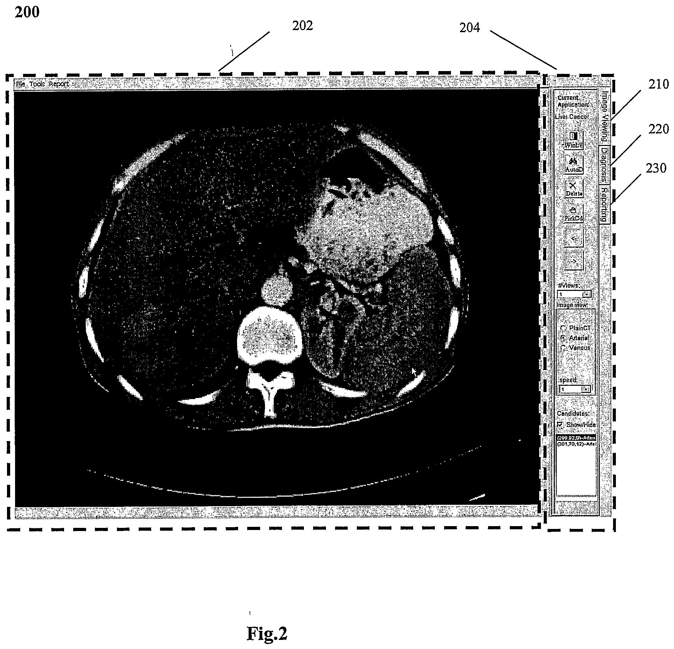

In some embodiments, a user may select, via a user interface (e.g., a visual data manipulation page), a particular data set to be visualized. A user may also choose to view the selected data in a particular manner, e.g., view data in its enhanced form, to improve the visual effect. A user may also activate a data processing tool through the user interface. A user may also control how a data processing tool is to be applied. For example, a data processing function may be applied to only a designated portion of the displayed data, which is determined, for instance, via a mouse click on a particular location of the display screen. Details related to various operations that can be effectuated through the data visualization/manipulation mechanism 114 are discussed with reference to Figs. 2-5.

In some embodiments, the automatic liver lesion detection mechanism 116 and the interactive liver lesion detection mechanism 118 provide capabilities to process a visual data set to detect liver lesion(s). Each may be activated under different circumstances. For example, the former may be activated when a user elects to perform automatic liver lesion detection without any user's intervention. The latter may be invoked when a user elects to interact with a detection process. In some embodiments, the automatic detection process may run concurrently with the interactive detection process with, for example, the automated process running as a backend process. The interactive detection process may run in the front end in real-time.

During an interactive lesion detection process, various types of interactions may be facilitated. For example, an interaction may include providing an indication, e.g., a bounding box drawn in an image, so that liver detection processing is applied to a restricted portion of a data set. Another example of such an interaction may relate to a confirmation dialog, in which the interactive liver lesion detection mechanism 118 may compute a confidence measure and/or various features characterizing each detected lesion and report such measure(s) to a user so that the user may decide either to accept or reject the underlying detection result based on an assessment of the provided measurements.

The automatic liver lesion detection mechanism 116 and the interactive liver lesion detection mechanism 118 may invoke the visual diagnostic information extraction mechanism 120 to extract one or more features associated with a detected liver lesion. Such features may include spatial and/or temporal features. For example, information related to a physical location, dimension, volume, or shape of an underlying lesion may be considered as spatial features. Patterns of intensity enhancement within a lesion over time may provide important diagnostic information in the temporal domain. Computation of such visual diagnostic information may be performed automatically or interactively. In some embodiments, such extracted measures may further be used to derive a confidence measure for each detected liver lesion. For instance, when there is no intensity enhancement over time in a lesion, it may provide an indication that the underlying lesion detected is unlikely to be a particular type, e.g., a malignant lesion. Features extracted by the visual diagnostic information extraction mechanism 120 may be used as visual diagnostic information.

In the exemplary construct of the system 100, the liver disease diagnosis mechanism 140 may function as an interactive diagnosis platform and facilitate different operations / interactions needed to reach a liver disease diagnosis based on diagnostic information obtained from different sources. In some embodiments, the liver disease diagnosis mechanism 140 comprises a hierarchical representation construction mechanism 122, a diagnostic evidence exploration / real time interactive diagnosis controller 124, and a pre-surgery assessment mechanism 126. The hierarchical representation construction mechanism 122 may integrate diagnostic information, visual and non-visual, that is made available by different sources, form a diagnostic information (DI) space by constructing one or more hierarchies of diagnostic information, and present such organized information via a graphical user interface (e.g., a liver disease diagnosis page) to assist a user to explore diagnostic information during an interactive diagnosis process. In some embodiments, a user may explore such organized diagnostic information by navigating through the DI space. A user may also interactively update the diagnostic information already extracted or add diagnostic information that has not been extracted.

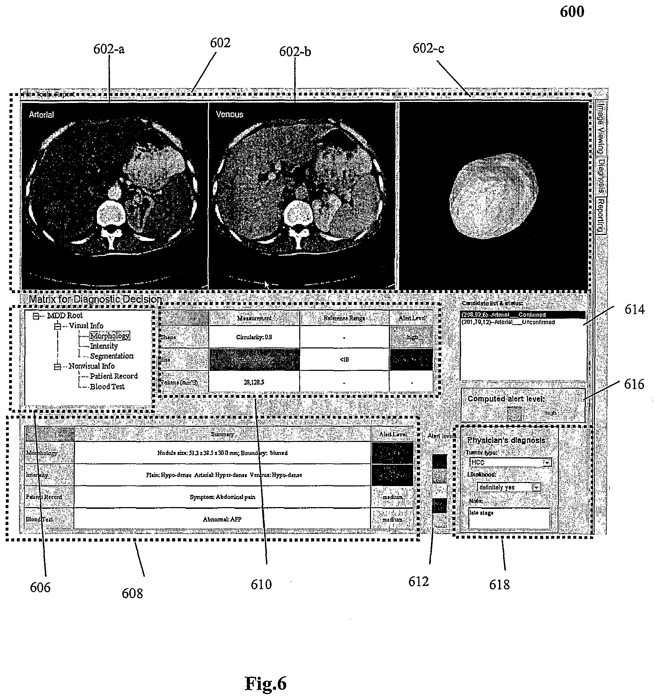

In some embodiments, the diagnostic evidence exploration / real time interactive diagnosis controller 124 may facilitate various interactive operations needed for a user to explore different types of diagnostic information, visual and non-visual, contained in a hierarchy and navigate through the diagnostic information space in order to reach a diagnosis. The pre-surgery assessment mechanism 126 may provide additional capabilities that facilitate different data visualization and manipulation to assist evaluation of the spatial relationship of different organic parts for the purposes of, e.g., surgical planning or treatment design. Details related to how an organized hierarchy of diagnostic information is presented and utilized to facilitate an interactive diagnosis process are discussed with reference to Figs. 6-12.

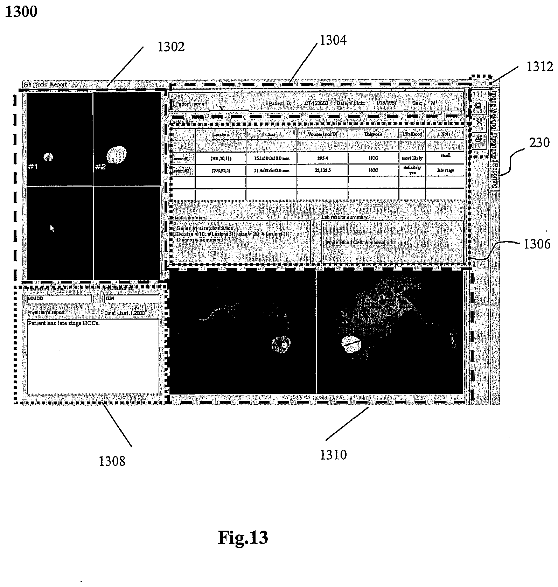

In some embodiments, a diagnostic result produced during the interactive diagnosis process may be used to generate a clinic report. This may be facilitated by the diagnostic report generation mechanism 128. Such a report may be automatically generated and interactively refined. For example, each lesion contained in an automatically generated report may require confirmation from a user in order to be included in a final clinic report. In some embodiments, such a clinic report may include visual views of each lesion detected, visual renderings showing the spatial relationship between a lesion and different anatomies it connects to (e.g., lobe, blood vessels, etc.), various features characterizing the lesion, medical diagnosis and/or assessment for each lesion, and/or one or more treatment plans derived based on the diagnosis. Details related to a clinic report generated based on a liver disease diagnosis are discussed with reference to Figs. 13-14.

Fig. 2 shows an exemplary layout of a data manipulation page 200, according to an embodiment of the present invention. Upon retrieving patient related and liver disease specific information (which may be visual and/or non-visual) from the patient database 106, a user may, through the exemplary data manipulation page 200, visualize selected information and/or extract additional diagnostic information by applying different manipulation operations. The data manipulation page 200 provides a graphical user interface through which various data visualization/manipulation operations may be effectuated. In some embodiments, the data manipulation page 200 comprises two main areas, a data visualization area 202 and a data manipulation control area 204. In the data visualization area 202, one or more visual data sets may be visualized. In the data manipulation control area 204, one or more selection means, where each of the selections may represent a graphical user interface for a different stage of the diagnosis process, may be provided that enable a user to make a selection or switch from one stage of the process to another. In the exemplary layout 200, there are three selectable interfaces, corresponding to an image viewing interface 210, a diagnosis interface 220, and a reporting interface 230. In this example, a selection on the image viewing interface 210 causes the display of the data manipulation page 200.

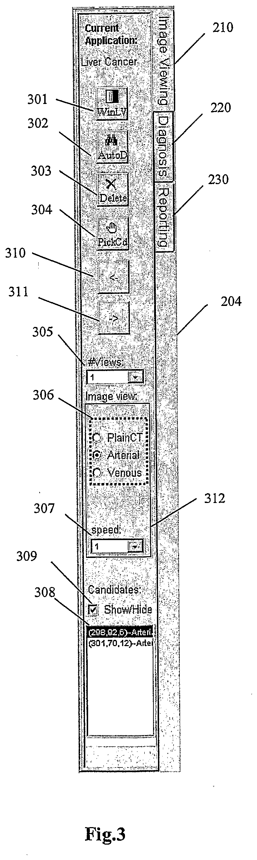

The data manipulation control area 204 on the data manipulation page 200 may also include a plurality of actionable visual means through which a user may exercise control in data visualization and manipulation. Fig. 3 shows an enlarged view of the layout of the data manipulation control area 204, according to an embodiment of the present invention. The exemplary data manipulation control area 204 includes an actionable button 301 for window display parameter control, an actionable button 302 for activating an automatic lesion detection operation, an actionable button 303 for deleting a selected lesion candidate, an actionable button 304 for activating an interactive lesion detection operation, a sub-window 305 with an activatable pull-down menu for entering a choice regarding a number of data sets to be visualized, a rendering control panel 312, a sub-window 308 for displaying a list of selectable lesion candidates detected, a clickable selection button 309 for controlling whether a selected lesion candidate is to marked up by, e.g., superimposing the boundary extracted from the lesion on the display of the image, and a pair of clickable buttons 310 and 311 for advancing slices of a visualized volume in a forward or a backward direction.

In some embodiments, the display parameter controllable through the actionable button 301 may include a contrast level parameter. For example, when the contrast within an image in the display is low, such an initial contrast range may be expanded to make the content in the image more visible by adjusting each pixel value proportionally against an expanded intensity range. The expanded intensity range may be a display control parameter. In some embodiments, the button 301 may be designed as a toggle button so that a user may click on the button 301 to a state in which the user may then use a mouse to control the level of contrast by, e.g., dragging the mouse upward to obtain a higher contrast level. Such produced changes to an intensity range may be applied in real time to the image in the display (e.g., the image displayed in the data visualization area 202) so that a user may observe the feedback and determine an appropriate level of contrast in action. Alternatively, a right click on the mouse may cause a pull-down menu to be displayed which may provide a user a list of selections of contrast level or a sliding bar that allows a user to slide (similar to a mouse dragging action) to select an appropriate contrast level. When an appropriate contrast level is found, the user may click on the button 301 again to toggle back to a state where the mouse is released for other purposes.

Other visualization control may also be provided. For example, a data set on display may be visualized using its enhanced version. In some embodiments, such an enhanced version of a data set may correspond to a liver intensity subtracted (LIST) image, derived by subtracting each pixel value using, e.g., an average intensity value of the liver. With such an LIST image, a lesion with enhanced intensity values may be visualized in a more effective way. LIST values may also be displayed as grey values or pseudo colors.

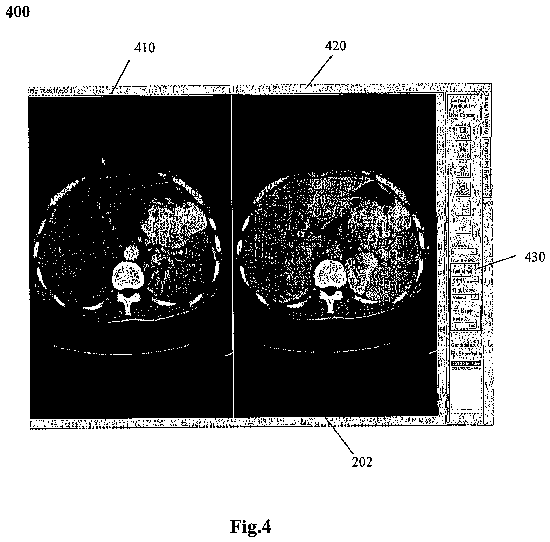

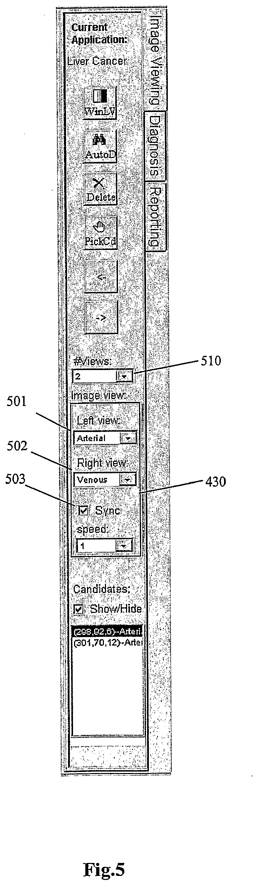

The actionable button 302 may be clicked to activate the automatic lesion detection mechanism 116 (see Fig. 1). Once activated, the automatic lesion detection mechanism 116 performs lesion detection with respect to a data set that may be set as an active data set. In some embodiments, when there is only one data set being visualized, it may be automatically considered as an active data set. When more than one data set is visualized (an exemplary scenario is discussed below with reference to Figs. 4 and 5), a selection of an active data set may be made through, e.g., a click in the display of a desired active data set. In some embodiments, the automatic lesion detection may be applied to the entire active data set. In some embodiments, the detection may be applied to a portion, or a region of interest, of an active data set. Such a region of interest may be defined in different ways. In some embodiments, a user may manually define such a region by, e.g., clicking at a location in a displayed image or drawing a bounding box in the displayed image. Such a clicked location may be used as a seed from where the detection operation is initiated. A bounding box may serve as a boundary of the detection operation so that only data within the bounding box is to be processed.

In some embodiments, during lesion detection, the interactive lesion detection mechanism 118 may display feedback information, e.g., in real time, in the image visualization area. Such feedback information may include one or more features computed with respect to a detected lesion. Examples of such features include spatial features such as an estimated dimension, volume, or shape of the detected lesion and/or temporal features such as intensity enhancement patterns over time. Such feedback information may also include an indication that there is no lesion detected or a confidence measure indicating the likelihood that there is a lesion at the location detected. The interactive lesion detection mechanism 118 and automatic lesion detection mechanism 116 may invoke the visual diagnostic information extraction mechanism 120 to compute various features. The interactive lesion detection mechanism 118 may be repeatedly activated when a user specifies a new region of interest. For example, a user may press the button 302 and then click at a first location in a displayed image. When the detection is completed at the first location, the user may click on a second location in the displayed image. In this case, the interactive lesion detection mechanism 118 may be invoked again to perform detection at the second location. In some embodiments, under an interactive lesion detection mode, each detected lesion may be automatically added to the list of lesion candidates displayed in the sub-window 308.

Each lesion candidate in the list may be selected from the sub-window 308 for visualization purposes. Data visualized in the data visualization area 202 may be automatically adjusted according to a selected lesion candidate. For example, the visualized slice in the data visualization area 202 may correspond to a slice that contains the selected lesion candidate. Through the sub-window 308, a user may also edit the list of lesion candidates. For example, a user may select a lesion candidate in the sub-window 308 (e.g., by clicking on it or highlighting it) and then delete the selected lesion from the list. The deletion may be effectuated by, e.g., pressing the delete button 303 or selecting a deletion operation from a pull-down menu displayed near the selected lesion displayed in the data visualization area 202 when, e.g., a right button of the mouse is clicked.

The rendering control panel 312 may comprise different control means, depending on whether one or more data sets are visualized in the data visualization area 202. In some embodiments, when only one data set is specified (e.g., in the sub-window 305) to be visualized, the data rendering control panel 312 may comprise a view selection region 306 and a speed control sub-window 307 with, e.g., a pull-down menu for entering a desired speed at which a data set is to be played. The selection region 306 may provide a list of visual data sets that can be visualized. For example, in Fig. 3, there are three exemplary data sets listed: a plain CT data set, a data set from an arterial phase, and a data set from a venous phase. Each of the data sets may be displayed with a clickable radio button through which a user may make an exclusive selection. When there are more data sets available than the space permits, there may be a scroll mechanism provided to allow a user to scroll up and down to make a selection.

A speed specified in the sub-window 307 may be used in controlling how fast to play 3D volumetric data. In some embodiments, the playing speed may be measured by a number of slices per second. There may be alternative means to control the speed of data play. For example, a user may use a mouse to control the play speed by holding-down a left button on the mouse and then dragging the mouse in, e.g., an upward direction, in order to advance the play, e.g., in a forward direction and the play speed may be adapted proportionally to the dragging speed. In some embodiments, the play speed may also be dynamically adjusted during a play according to some meaningful event. For example, the play speed may be automatically slowed down when the displayed slice is within a certain distance of a detected lesion. Such an automated speed control may help a viewer to pay more attention to a lesion region. Other means to call for a user's attention may include displaying a text string on the display screen or producing an alert sound to warn the user. Such means to control the advancing slices in a 3D data may differ from the function achieved by the clickable buttons 310 and 311, where each click may advance one slice and provide a manual play speed control.

In some embodiments, the number of data sets to be visualized in the data visualization area 202 may be configured through the sub-window 305. For example, images acquired at different phases may be viewed simultaneously in the data visualization area 202. Such different data sets may be displayed side by side or when they are played, as a movie, they may be controlled to be played synchronously or asynchronously. Fig. 4 shows a different exemplary layout 400 of the data manipulation page 200, according to an embodiment of the present invention. In this exemplary layout, there are two data sets visualized in two sub-areas, 410 and 420 of the data visualization area 202. When more than one image is visualized, the rendering control panel 312 may accordingly provide different control capabilities as shown in 430.

Fig. 5 shows an exemplary layout of the rendering control panel 204, according to an embodiment of the present invention. In the sub-window 510, it is specified that two data sets be visualized in the data visualization area 202. Based on that, there are corresponding two sub-windows 501 and 502, each of which may be provided to facilitate selection of a data set to be visualized in a left view and a right view, respectively. In the example shown in Fig. 4, the left view corresponds to a CT data set acquired at an arterial phase and the right view corresponds to a related CT data set acquired at a venous phase. In addition, a clickable button 503 may be provided that enables a user to indicate whether the two data sets are to be visualized in a synchronous or an asynchronous manner.

In some embodiments, when two data sets are displayed side by side synchronously, the corresponding slices displayed in the left and right view at the same time may be registered. Such a registration may be performed based on different criteria. For example, a registration may be based on the coordinates of each 3D slice in each of the volumes. Using CT data as an example, two views displayed side by side may correspond to slice images at a same physical location of a subject (e.g., a patient) but acquired at different times. When there is a lesion present, such two views may reveal how the lesion has changed over time. For example, in CT modality, corresponding slice images from the plain CT phase, the arterial phase, the portal venous phase, and the delayed phase may all be displayed at the same time. By so doing, a lesion in one phase may be compared with the same lesion in other phases to allow visualization of contrast enhancement patterns, which may be helpful in reaching a diagnosis decision.

In some embodiments, a user may also manually register the views in each display window. For instance, a user may unclick the button 503 (e.g., to unlock the registration) and then use the button 310 or 311 to advance, in one of the data sets (e.g., by setting that data set as an active one) slice by slice until the user determines that the two views on the display actually correspond to each other. At this point, the user may click the button 503 to make the two views registered.

When more than one data set is visualized, slice images active in the data visualization area 202 that are from separate data sets may be interpolated to produce a new slice view. Intensity change in a lesion with respect to normal liver tissue across different image phases is often an important feature used in detecting a lesion. Registered slices (e.g., registered based on 3D coordinates of the slices) in different phases/times may be interpolated in the time domain to form an extended slice sequence. Such an extended sequence may be visualized, e.g, as an animated movie, providing diagnostically useful information related to a continuous intensity change in the time domain.

In some embodiments, when more than one data set from a time sequence is accessible, an enhanced version of the data sets may be generated based on LIST images. As described earlier, a LIST image is derived by subtracting, e.g., an average intensity of the liver and such an image may better reveal the presence of a lesion. By subtracting corresponding LIST images of different data sets in a time sequence such as data acquired at different phases, this may produce enhanced intensity change of a liver lesion over time. Such generated subtracted LIST images may provide useful diagnostic information and may be visualized using, e.g., grey-levels or pseudo-colors.

In some embodiments, different types of diagnostic information derived via the visual data manipulation mechanism 130 may be utilized, by the liver disease diagnosis mechanism 140, to assist a user in reaching a diagnostic decision. Fig. 6 shows an exemplary layout of a liver disease diagnosis page 600, according to an embodiment of the present invention. The exemplary liver disease diagnosis page. 600 comprises a visual data viewing area 602, a hierarchical representation display area 606, a diagnostic information display area 610, a diagnostic information summary display area 608, a lesion candidate display area 614, a diagnosis panel 618, an overall alert level display panel 616, and an alert level reference chart 612.

In some embodiments, given a lesion candidate, selected either by clicking a specific item in a list of lesion candidates (displayed in the lesion candidate display area 614) or by clicking on a graphic overlay of a lesion candidate mark on an underlying image, a liver disease diagnosis page with respect to the selected lesion may be generated. In such a diagnostic page, different types of diagnostic information may be integrated or fused, either automatically or semi-automatically, and organized into one or more hierarchies to be presented to a user so that the user may explore different information in a hierarchy or a diagnostic information (DI) space. The hierarchy of diagnostic information may include both visual and non-visual. Visual diagnostic information may include a lesion size, a lesion location in a liver lobe, an indication of whether a lesion is on the liver peripheral edge, or an intensity enhancement pattern extracted from different phases of liver imaging. Non-visual diagnostic may include liver-disease-specific information extracted from a patient record, relevant lab test results, or genotype information.

In some embodiments, to visualize the diagnosis page, a user may click on area 220 (see Fig. 2) to make the diagnosis page be in an active view. Such a diagnosis page may be rendered upon being activated. The diagnostic evidence exploration and real time interactive diagnosis controller 124 may render the diagnosis page and control the interactions between a user and the diagnosis page. In the exemplary layout of the diagnostic page (as seen in Fig. 6), the visual data viewing area 602 may be used to display visual diagnostic information. For example, there may be a certain number of original slice images on the display that, e.g., contains the selected lesion. As illustrated in Fig. 6, sub-regions 602-a and 602-b in Fig. 6 display two slice images from, e.g., different phases. Detected lesions may be marked in such views (not shown). The number of views on the display may be defined, for example, by a user in the data manipulation page (see sub-window 430 in Fig. 5). The visual data viewing area 602 may also comprise a 3D rendering of the selected lesion in its segmented form, as shown in sub-area 602-c. This 3D rendering of the segmented lesion may be manipulated for visualization or diagnosis purposes. For instance, a user may rotate or translate the 3D rendered object through, e.g., a mouse drag movement. A user may also zoom in and out of a particular portion of the 3D lesion. A user may also display a 3D vessel structure and a liver volume onto a same view to, for example, to obtain an improved perception of the spatial relationship. In some embodiments, a user may also insert a plane at an arbitrary location and orientation in the 3D rendering space to intersect the 3D lesion so that a cross section of the lesion on the plane may be visualized in the sub-region 602-a or 602-b. Such data manipulation operations may be effectuated through different mouse actions, such as click coupled with pull-down or pull-up menus.

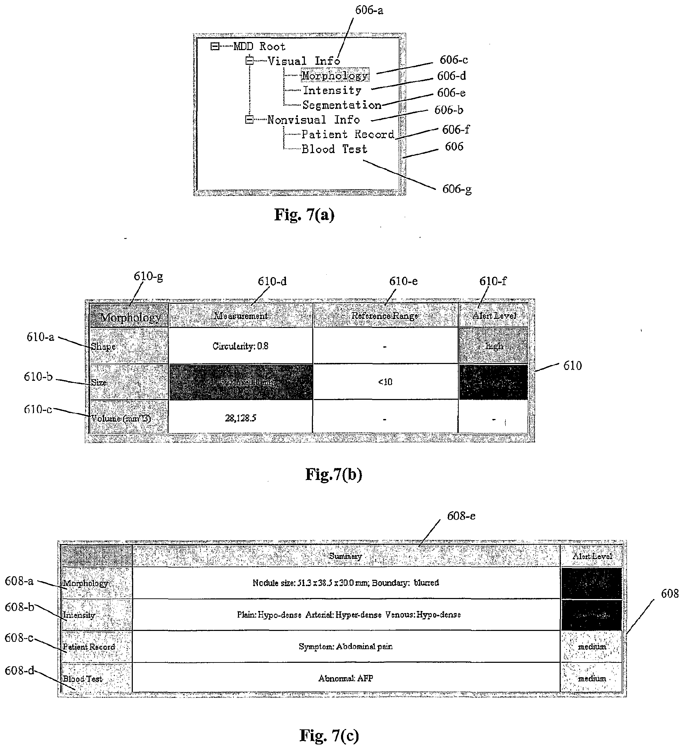

Non-visual diagnostic information associated with the selected lesion may also be viewed and explored via a hierarchical representation of diagnostic information, constructed with respect to the selected lesion candidate. Fig. 7(a) illustrates an enlarged view of the hierarchical representation display area 606, according to an embodiment of the present invention. In this exemplary hierarchy, diagnostic information is organized as groups, each corresponding to a specific type of diagnostic information. For example, the exemplary hierarchy representation, as shown in Fig. 7(a), organizes information into visual (606-a) and non-visual (606-b) diagnostic information categories. The visual diagnostic information (606-a) comprises sub-categories of diagnostic information such as morphologic diagnostic information 606-c, intensity related diagnostic information 606-d, and segmentation related diagnostic information 606-e. Similarly, non-visual diagnostic information 606-b may also be further organized into sub-categories, such as diagnostic information from a patient record (606-f) and diagnostic information from various lab tests, such as blood test 606-g. In some embodiments, any category or sub-category may be selected for further examination. This may be effectuated by a mouse click, which may cause the clicked category to be highlighted (as shown in Fig. 7(a) on the category "Morphology").

In the exemplary interface shown in Fig. 6, information displayed in different areas of the page may be coordinated and updated in a coordinated fashion on the fly when a user selects a different piece of information for examination. For example, when a lesion candidate is selected by clicking on the list of lesion candidates (displayed in the lesion candidate display area 614) or clicking on a lesion in an image, visual diagnostic information associated with the selected lesion may be visualized in the area 602, which may include both original slice images displayed in 602-a and/or 602-b, with or without superimposed lesion segmentation, and a 3D rendering of the segmented lesion in 602-c. Through a hierarchical representation of diagnostic information related to the selected lesion displayed in the hierarchical representation display area 606, a user may elect to explore any piece of non-visual diagnostic information. In some embodiments, when a user selects a specific category of non-visual diagnostic information via the hierarchical representation, the selected category of diagnostic information may be displayed in the diagnostic information display area 610. A summary with respect to all categories of non-visual diagnostic information may be presented in the diagnostic information summary display area 608.

Fig. 7(b) illustrates an exemplary tabular form of the diagnostic information display area 610, according to an embodiment of the present invention. In this exemplary layout, each row may correspond to a feature within a selected category of non-visual diagnostic information and each column may correspond to a particular aspect associated with a feature of the selected category. For example, when the DI category of "Morphology" is explored, the tabular constructed for the morphology category (610-g) may include features related to the morphology of the selected lesion candidate such as estimated shape (610-a), size or dimension (610-b), or volume (610-c) of the lesion candidate. Each of such included features may be described in terms of different aspects of information that may be relevant to a diagnosis. For example, a measurement made with respect to each feature (610-d), an acceptable or reference range for the feature (610-e), or an alert level (610-f) estimated, e.g., automatically, based on each feature measurement and some given knowledge. The display of non-visual diagnostic information may be coupled with color codes to produce some desired visual effect. For example, for a feature that has a value suspiciously out of a corresponding acceptable/reference range, the display area for the feature value may be color coded as, e.g., red to produce an alarming or warning effect. A similar color coding scheme may also be applied to the display area for an estimated alert level (610-f). Content displayed in the diagnostic information display area 610 may be updated dynamically when a user switches to a different piece of diagnostic information during exploration.

Fig. 7(c) illustrates an exemplary tabular layout for the diagnostic information summary display area 608, according to an embodiment of the present invention. In this exemplary layout, each row may represent a distinct category of diagnostic information and each column may provide a summary or evaluation with respect to each category of diagnostic information. Each row in the exemplary table illustrated in Fig. 7(c) may correspond to a category of diagnostic information listed in the hierarchical representation display area 606. For example, given four categories of non-visual diagnostic information in the hierarchical representation in Fig. 7(a), the diagnostic information summary display area 608 may include corresponding four rows, a row for "Morphology" DI (608-a), a row for "Intensity" DI (608-b), a row for DI from a "Patient Record" (608-c), and a row for DI from "Lab Tests" (608-d). For each category of DI, a summary (608-e) of all DI from that category may be provided. In addition, an evaluation may also be provided such as an alert level estimated based on, e.g., the overall diagnostic information in the underlying category and certain given knowledge. Such evaluation may be performed automatically and may be displayed coupled with some color coding scheme to produce an appropriate warning effect.

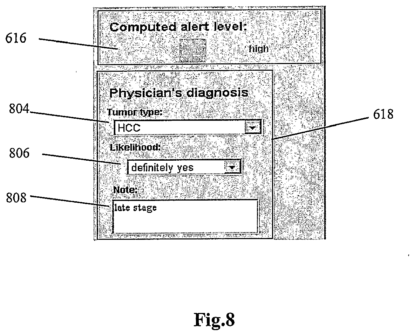

In some embodiments, based on displayed visual and non-visual diagnostic information explored in an interactive diagnosis process, a user may interact with the diagnosis panel 618. Fig. 8 shows an exemplary layout of the diagnosis panel 618, together with the overall alert level display panel 616, according to an embodiment of the present invention. In this exemplary layout, the diagnosis panel 618 comprises a diagnosis decision window 804, a confidence measure window 806, and a diagnostic comments window 808. In some embodiments, a diagnosis decision and/or information related to such a diagnostic decision may be automatically computed and displayed, e.g., as default. For example, an automatically derived diagnosis decision, e.g., a lesion is of HCC type, may be displayed in the diagnosis decision window 804. A confidence measure automatically derived based on all available diagnostic information and some knowledge, may be displayed in the confidence measure window 806. An evaluation related to, e.g., a development stage of an underlying lesion, may be displayed in the diagnostic comments window 808.

In some embodiments, one or more pieces of information displayed in windows 804, 806, and 808 may be interactively or manually changed. For example, a user may enter a revised diagnosis in window 804. This may be achieved by typing in a diagnosis in window 804 or by selecting a known type of liver disease in a pull-down menu activated by clicking on the pull-down menu button in the window 804. Similarly, a confidence measure and/or comments associated with a diagnosis may also be modified by a user in corresponding windows 806 and/or 808.

In some embodiments, through the hierarchical representation, a user may navigate in an organized hierarchical information space via operations (e.g., a mouse movement or a mouse click) performed through the graphical user interfaces as described herein. Different types of diagnostic information may be explored through effective navigation in the DI space. Navigation may proceed in any desired order. For example, a user may navigate between different levels of diagnostic information in the hierarchy. Whenever a user navigates to a different subspace in the hierarchy, the liver disease diagnostic page 600 may be dynamically reconfigured. In some embodiments, a data exploration process or a navigation path associated with a user may be made visible to the user or may be recorded so that the user may revisit or reexamine a diagnostic decision making process.

In some embodiments, a hierarchical representation of diagnostic information may organize diagnostic information with respect to disease types. In such an organization, a hierarchical tree may be employed, in which leaves may represent corresponding liver disease types. Each non-leaf node may correspond to a piece of diagnostic information and a link between two nodes (or between a node and a leaf), if any, may correspond to a specified condition that has to be satisfied in order to transit from the node at a higher level to the node at a lower level in the tree. In some embodiments, a user may utilize such a tree structure to interactively explore different pieces of diagnostic information and to reach a diagnostic decision. For example, a user may initiate the diagnostic process by starting from a root node of the tree and following appropriate links to complete different transitions based on a given set of diagnostic information. When the user reaches a leaf of the tree, a diagnostic decision may accordingly be concluded.

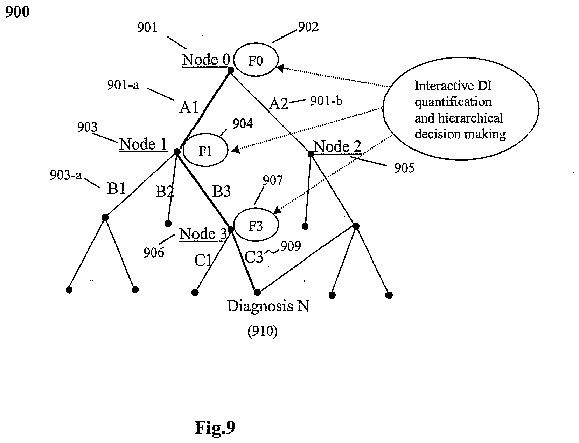

Fig. 9 shows an exemplary hierarchy of diagnostic information organized as a tree 900, according to an embodiment of the present invention. In this exemplary tree 900, there are a number of nodes (e.g., Node 0 901, Node 1 903, Node 2 905, Node 3 906) where Node 0 901 may represent a root or a starting node. At each node, there may be one or more pieces of diagnostic information needed. For instance, at the Node 901, a user may access a piece or a set of diagnostic information denoted by F0 (902) in order to determine what transition is possible in the tree. At Node 1 903, diagnostic information F1 904 is needed to make a similar decision. At Node 3 906, diagnostic information F3 907 is needed. Between every two connected nodes, there may be a condition associated with a link between the two nodes that defines certain circumstances under which a node at a higher level may traverse to the connected node at a lower lever. For example, one may traverse from the Node 0 901 to the Node 1 903 when the diagnostic information F0 902 satisfies a condition A1 901-a. Similarly, one may traverse from the Node 1 903 to the Node 3 906 if the diagnostic information F1 904 satisfies a condition A2 901-b. In this example, to reach a diagnosis decision N 910, a collection of diagnostic information (e.g., F0 902, F1 904, and F3 907) has to satisfy a number of conditions (e.g., A1 901-a, B3 903-b, and C3 909).

In some embodiments, an evaluation as to whether a condition along a link between two nodes is met may be performed as an exact match. In other embodiments, such an evaluation may be made based on an inexact match. In this case, a similarity measure between a piece of relevant diagnostic information and a corresponding feature used to define a condition for a transition may be computed. An inexact match may be effectuated by, e.g., assessing whether the similarity measure exceeds a certain threshold. If the similarity exceeds the threshold, the condition may be considered to be met and the transition may take place.

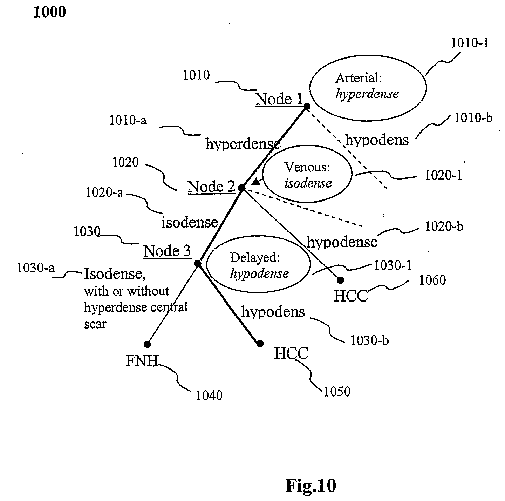

Fig. 10 shows an exemplary diagnostic tree 1000 for different types of liver diseases, according to an embodiment of the present invention. At the leaf level of this exemplary tree 1000, there are a number of liver diseases, each of which may require different types of diagnostic information extracted from different phases of CT images to satisfy some defined conditions. In this example, at Node 1 1010, diagnostic information 1010-1 that indicates that the underlying lesion is of a hyperdense type (1010-a), e.g., abbreviated as "arterial:hyperdense" is used to determine what transition is to take place. In this example, diagnostic information 1010-1 satisfies a condition 1010-a associated with the link between Node 1 1010 and Node 2 1020 so that a transition from Node 1 1010 to the Node 2 1020 takes place and the diagnostic process may now proceed to the Node 2 1020. At Node 2 1020, diagnostic information 1020-1 that indicates that the underlying lesion detected from an image at the venous phase is isodense (e.g., denoted as "venous: isodense") is used to determine the next transition. As a condition 1020-a (isodense) between the Node 2 1020 and a Node 3 1030 is satisfied, the diagnostic process may then make a transition from the Node 2 1020 to the Node 3 1030. At the Node 3 1030, diagnostic information 1030-1 that indicates that the underlying lesion shows hypodense property in the delayed phase image is used to make a determination whether the underlying lesion is either disease FNH 1040 or disease HCC 1050. As the diagnostic information 1030-b satisfies condition "Hypodense" defined on a link between the Node 3 1030 and the HCC 1050, a diagnosis decision is reached. This exemplary diagnostic tree illustrates how a collection of diagnostic information "arterial-hyperdense, venous-isodense, and delayed phase-hypodense" may give rise to a diagnosis of liver disease Hepatocellular Carcinoma (HCC) (rather than other diseases such as Focal Nodular Hyperplasia (FNH)).

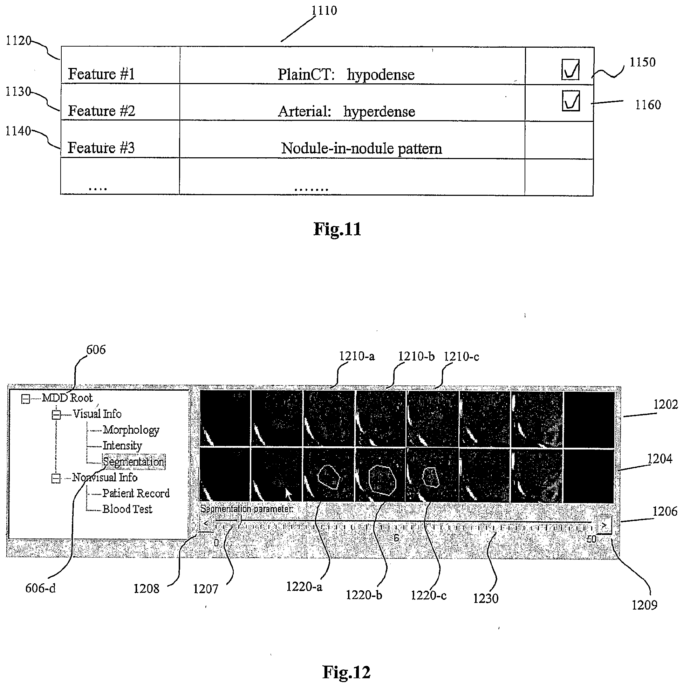

In some embodiments, a hierarchy of diagnostic information organized according to disease types may enable a user to navigate in the DI space through specific disease types. When a user specifies a disease type (e.g., HCC) while examining a selected lesion candidate, diagnostic information associated with the selected lesion may be displayed in such a manner that there is an indication for each displayed piece of diagnostic information as to whether this piece of diagnostic information satisfies one of the diagnosis criteria with respect to the specified disease type. Fig. 11 shows an exemplary tabular display 1110 of diagnostic information with indication of match against a specific disease type, according to an embodiment of the present invention. In this example, three pieces of diagnostic information (feature 1 1120, feature 2 1130, and feature 3 1140) are displayed with description thereof included in the table 1110. Two out of three features satisfy the diagnostic criteria of an underlying disease type. Such matches are indicated in the checkboxes 1150 and 1160.

In some embodiments, diagnostic information may be automatically extracted and incorporated in a corresponding hierarchical information space. A diagnostic decision may also be automatically made based on a collection of diagnostic information. In certain situations, a user may be provided with means to modify such automatically extracted diagnostic information via an operation through, e.g., a graphical user interface. For example, a user may add, delete, or revise diagnostic information by retyping a revised property associated with a piece of diagnostic information. When a change is made to a piece of diagnostic information, a diagnostic decision made previously based on this piece of information may be accordingly modified based on the updated diagnostic information. For example, using the diagnostic tree 1000 in Fig. 10 as an example, if a user changes the diagnostic information 1030-1 from "delayed: hypodense" to "delayed: isodense", the previous arrived diagnosis of HCC 1050 may be automatically changed to a new diagnosis of FNH 1040. This is because the revised diagnostic information "delayed: isodense" makes it impossible to transit from the Node 3 1030 to 1050. Instead, the revised description of a relevant property of the underlying lesion now satisfies the transition condition 1030-a between the Node 3 1030 and the diagnosis conclusion FNH 1040. As another example, when a user changes the description for, e.g., feature 1 1120 displayed in the table 1110 as illustrated in Fig. 11, the checkbox 1150 may be automatically unchecked if the revised diagnostic property does not satisfy a specified disease type.

In some embodiments, diagnostic information may also be modified through real time data processing using some data manipulation tools. Such tools may be interactive or automatic. In some embodiments, some data manipulation tools may be embedded with a piece of diagnostic information and may be activated when the piece of diagnostic information is being explored or on the display. For example, some real time interactive data processing tools may be embedded with a lesion detection result included in a hierarchical diagnostic information space. Such a lesion detection result may be visual (e.g., a boundary of a lesion) or non-visual (e.g., size or volume of a lesion) and different data processing tools may be individually embedded with appropriate diagnostic information. For example, a lesion segmentation tool may be embedded with an extracted lesion boundary. A spatial feature measurement tool may be embedded with a size estimated from a lesion. A visualization tool may be embedded with a visual data set from which a lesion is detected. In some embodiments, buttons or actionable icons may be embedded in table cells (e.g., for non-visual diagnostic information) or tree nodes in a hierarchical representation of diagnostic information.

Fig. 12 shows an exemplary interface of applying an embedded data manipulation tool 1206 to modify diagnostic information, according to an embodiment of the present invention. In this illustration, diagnostic information "Segmentation" 606-d is selected for exploration and a plurality of segmentation results in corresponding slice images may be visualized. For example, in an area 1202, regions of interest (ROI) slice images (e.g., 1210-a, 1210-b, and 1210-c) related to a lesion may be displayed. In an area 1204, these ROI slice images are shown with segmented lesion boundary superimposed (e.g., 1220-a, 1220-b, and 1220-c). A visual representation of an embedded interactive segmentation tool 1206 may be rendered that provides a sliding bar 1230. In some embodiments, the sliding bar 1230 associated with an interactive segmentation tool may include a clickable button 1208 at one end, a clickable button 1209 on the other end, and a sliding handle 1207. The button 1208 may be clicked to decrease the value of the operational parameter while the button 1209 may be clicked to increase the value of the operational parameter. The sliding handle may be moved along the sliding bar through, e.g., a mouse drag movement, to either increase or decrease the operational parameter. One example of such a segmentation operational parameter is a threshold used in determining the boundary of a lesion.

In some embodiments, when the operational parameter is adjusted, the segmentation result in display may be updated on the fly to show the effect. In addition, a chain reaction triggered by an updated segmentation may be automatically effectuated. Any measurement or decisions that are derived based on the segmentation result may accordingly be adjusted. For example, the size and volume of the underlying lesion may be updated on the fly. Furthermore, related decisions such as a confidence indicating a likelihood of a lesion or a diagnostic decision as to disease type may also be revised based on the updated segmentation information.

In some embodiments, tools for various data manipulations may be provided on the liver disease diagnosis page 600 (not shown). In some cases, such tools may be directly embedded with data itself. Examples of such tools may include automatic anatomy segmentation, 3D editing, and interactive visualization. Such tools may facilitate, e.g., segmentation of liver lobes and vessels, means for manual editing of segmented liver lobes and vessels (e.g., in a 2D or a 3D space), means to map segmented lesion, liver lobes, and vessels to a same reference frame, means to visualize and/or assess spatial relationships among lesions, vessels and lobes, etc. In some embodiments, a lesion may be rendered together with connected vessels and lobe fissures. With an appropriate tool that allows 3D rotation and translation, a user may be able to perceive 3D spatial relationship among different anatomical structure. This may provide effective assistance to perform, e.g., pre-surgical assessment of different lesions to derive, e.g., a treatment design or a surgical plan.

In some embodiments, tools may be provided to allow segmented liver lobes to be pulled apart electronically in 3D space while the spatial relationship among lesion(s) and vessels remain intact within each lobe. In one embodiment, this may be achieved by deriving a separate sub-volume for each type of anatomical structure (e.g., liver, lesion, and vessels) based on lobe boundaries. Each of such derived sub-volumes may be rendered independently. For example, the vessel sub-volume in the lobe segment III may be rendered as a different object as that in lobe segment IV. In some embodiments, a same object-to-view transformation may be assigned to all sub-volumes that lie within the same lobe segment. When a mouse location is within the proximity of a lobe boundary, all sub-volumes within the lobe segment may become activated. Whenever a sub-volume becomes active, a mouse motion around the active sub-volume may be translated into a motion to be applied to the sub-volume. A motion to be applied to a sub-volume may be a rotation, a zooming action, a translation, or a combination thereof. When such a motion is applied to all sub-volumes within the same lobe, it may create a visual effect that all objects within the same lobe move simultaneously as one body. In this exemplary scheme, each liver lobe may be individually manipulated and visually examined. In some embodiments, such a manipulation may allow a user to more precisely assess the spatial relationship between different anatomical structures, which may provide important diagnostic information for treatment design or surgical planning. In some embodiments, different lobes and sub-volumes may be reassembled to form a unified entity via, e.g., a mouse click.

In some embodiments, a clinical report associated with one or more lesions may be automatically generated. Such a clinical report may incorporate different types of information and provide automatically summarized medical evidence that support each diagnosis. A clinic report may also include information obtained, e.g., through a pre-surgery assessment process such as spatial features of each lesion, the spatial relationship between a lesion and its nearby anatomical objects, measurements to be used in a surgery plan, or different hypotheses as to how the surgery may be performed with respect to each lesion. In some embodiments, snapshots of each liver lesion, either as a set of 2D region-of-interest (ROI) sub-images across different phases or as a 3D rendered view, may also be automatically incorporated in a clinical report. Non-visual diagnostic information, such as a blood test or family history of liver disease, may also be automatically summarized and incorporated. In some embodiments, a user may be provided with means to enter a diagnosis report, including a treatment plan or a surgical plan.

Fig. 13 shows an exemplary layout of a reporting page 1300, according to an embodiment of the present invention. In this example, a clinic report comprises a patient information portion 1304, a lesion diagnostic information summary portion 1306, a comments portion 1308, a 3D lesion display portion 1302, and an internal anatomy visualization portion 1310. In this illustration, the 3D lesion display portion 1302 includes two snapshots of the 3D rendering for the two detected lesions (which are listed in the lesion summary portion 1306). In some situations, when more lesions are detected, the size of each snapshot may be automatically reduced.

In some embodiments, the internal anatomy visualization portion 1310 may include a plurality of views that reveal the spatial relationship among different objects (liver, lesions, vessels). In this example, the internal anatomy visualization portion 1310 includes two views. The image visualized on the right in 1310 may correspond to a 3D rendering of a lesion together with connected vessels and other anatomical structures, as they exist in reality. The image visualized on the left side in 1310 may correspond to an electronic rendering of a pulled-apart liver (e.g., the front half is ripped away) with the internal spatial relationship between a lesion and its surrounding vessels revealed.

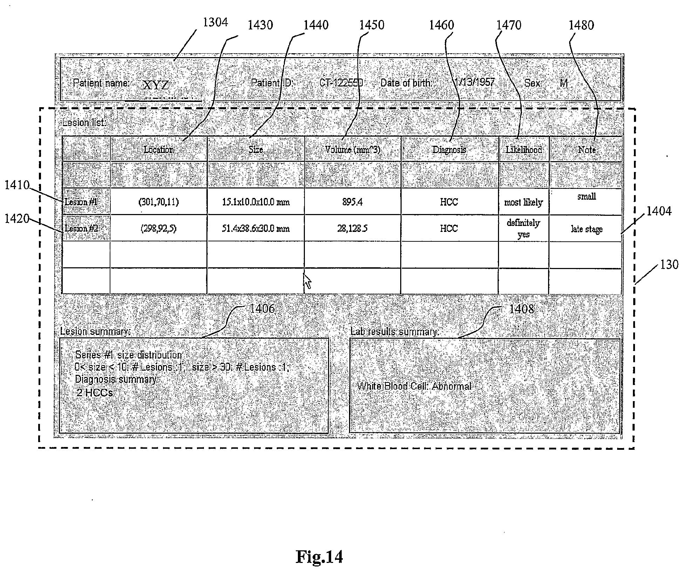

Fig. 14 shows an exemplary layout of the lesion diagnostic information summary portion 1306, according to an embodiment of the present invention. In the exemplary layout for the summary portion 1306, there is a lesion list sub-area 1404, a lesion summary sub-area 1406, and a lab result summary sub-area 1408. In this example, each row in the lesion list sub-area 1404 may correspond to a lesion detected (e.g., two lesions corresponding to 1410 and 1420) and each column may provide different descriptions for a particular property associated with a lesion. For instance, a plurality of property descriptions are provided, including a location description 1430, a size description 1440, a volume description 1450, a diagnosis associated with a lesion 1460, a likelihood characterization 1470, and a note made for each lesion 1480.

## Claims

1. A system for liver disease diagnosis, comprising:
a data retriever capable of retrieving one or more liver visual data sets and non-visual information associated with a subject and specific to a liver disease; and
a visual data manipulation mechanism capable of:
(i) rendering a visual data manipulation page, wherein the visual data manipulation page includes:
a first area for displaying images of the one or more data sets and for manipulating the one or more data sets, and
a second area for providing a plurality of selectable means to activate one or more data manipulation operations to be performed with respect to the one or more data sets displayed in the first area,
wherein, when the first area is configured to manipulate more than one data set, each image is from a corresponding data set and images from different data sets can be displayed synchronously;
(ii) visualizing one or more retrieved liver visual data sets in the visual data manipulation page, and
(iii) effectuating one or more data manipulation operations, activated via the plurality of selectable means displayed on the visual data manipulation page and to be performed with respect to the one or more data sets, wherein more than one data set can be visualized in a synchronized manner,
wherein a speed of displaying different slices of a data set of volumetric data in the first area is determined based on a distance of a displayed slice from a lesion candidate slice.

2. The system according to claim 1, wherein a liver visual data set includes an image and/or a volume acquired in an imaging modality.

3. The system according to claim 2, wherein the imaging modality includes at least one of computerized tomography (CT), magnetic resonance imaging (MRI), and positron emission tomography (PET).

4. The system according to claim 3, wherein CT liver data comprises data of different phases including at least one of a plain CT phase, an arterial phase, a venous phase, and a delayed phase.

5. The system according to claim 4, wherein the more than one images displayed in the first area are images from different CT phases.

6. The system according to claim 1, wherein the non-visual information includes information contained in a medical record of the subject.

7. The system according to claim 1, wherein the non-visual information includes a lab test result associated with the subject.

8. The system according to claim 1, wherein the non-visual information is specific and relevant to the liver disease type.

9. The system according to claim 1, wherein the plurality of selectable means include at least one of:
a clickable icon configured for advancing, in a first direction, in a volumetric data set to select a slice to be displayed in the first area;
a clickable icon configured for advancing, in a second direction, in the volumetric data set to select a slice to be displayed in the first area;
a clickable icon configured for adjusting a display parameter associated with a data set displayed in the first area;
a clickable icon configured for activating a process of automatic lesion detection applied to a data set displayed in the first area;
a clickable icon configured for activating an interactive process of lesion detection applied to a data set displayed in the first area;
a sub-area configured for facilitating a specification of one or more data sets to be displayed in the first area;
a sub-area configured for manipulating information associated with a list of lesion candidates detected from a data set displayed in the first area; and
a clickable icon configured for deleting information associated with a lesion candidate included in the list of lesion candidates.

10. The system according to claim 9, wherein a clickable icon is a button.

11. The system according to claim 9, wherein the sub-area for specifying a data set to be displayed comprises:
a window for entering a selectable number indicating the number of data sets to be displayed in the first area; and
one or more areas, each corresponding to a sub-area in the first area in which a data set is to be displayed, facilitating specification of a data set to be manipulated in the corresponding sub-area in the first area.

12. The system according to claim 11, further comprising, when more than one data set is to be displayed, an icon through which a mode of display across different data sets is defined.

13. The system according to claim 12, wherein the mode of display across different data sets includes one of a synchronized mode and an asynchronized mode.

14. The system according to claim 9, wherein the sub-area for manipulating information associated with a list of lesion candidates comprises:
a window area in which the information associated with a list of lesion candidates is displayed;
a clickable icon configured for facilitating control of overlay of the information associated with the list of lesion candidates in the first area where the underlying data set is visualized.

15. The system according to claim 9, wherein an adjustment to the display parameter can be effectuated via a mouse movement/click when the clickable icon for adjusting the display parameter is clicked.

16. The system according to claim 9, wherein visual information to be displayed in the first area is an enhanced version of a data set, and wherein the enhanced version of a data set includes one or more liver intensity subtracted (LIST) images.

17. The system according to claim 16, wherein the enhanced version of a data set includes one or more images derived by subtracting adjacent LIST images.

18. The system according to claim 9, wherein visual information to be displayed in the first area is an enhanced version of a data set, and wherein the enhanced version of a data set is obtained by interpolating more than one registered slice of corresponding more than one data set acquired at different times, and the interpolated slice is displayed as an animated movie.

19. The system according to claim 9, wherein the system is arranged to perform the automatic lesion detection as a backend process.

20. The system according to claim 9, wherein the system is arranged to perform the interactive lesion detection with respect to a marking created based on a visualization of a data set in the first area.

21. The system according to claim 20, wherein the system is arranged to perform the automatic and the interactive lesion detection processes to extract one or more features associated with a detection result.

22. The system according to claim 21, wherein the one or more features include at least one of:
a likelihood value indicating how likely it is that a lesion is present near the marking; and
at least one measurement made with respect to each lesion detected.

23. The system according to claim 1, wherein the visual data manipulation mechanism is further capable of rendering a liver disease diagnostic page, which includes at least one of:
a first region configured for displaying a list of selectable lesion candidates detected from at least one data set;
a second region configured for displaying visual information related to a lesion candidate selected from the list of selectable lesion candidates;
a third region configured for displaying diagnostic information associated with the selected lesion candidate; and
a fourth region configured for recording information related to a diagnosis with respect to the lesion candidate selected.

24. The system according to claim 23, wherein the second region comprises one or more sub-regions, in each of which a data set or the selected lesion candidate can be visualized and/or manipulated.

25. The system according to claim 23, wherein the third region further comprises:
a first sub-region configured for displaying a hierarchical representation of selectable diagnostic information related to the selected lesion candidate;
a second sub-region configured for displaying a type of diagnostic information selected from the hierarchical representation with respect to the lesion candidate selected;
a third sub-region configured for presenting a summary of the diagnostic information in the hierarchical representation that is associated with the lesion candidate selected; and
a fourth sub-region configured for displaying information related to an overall level of alert with respect to the lesion candidate selected.

26. The system according to claim 25, wherein the hierarchical representation includes visual and/or non-visual diagnostic information.

27. The system according to claim 25, wherein the hierarchical representation organizes information based on diagnostic information types.

28. The system according to claim 25, wherein the hierarchical representation organizes information based on liver disease types.

29. The system according to claim 25, wherein each piece of the selectable diagnostic information in the hierarchical representation can be selected through a mouse click.

30. The system according to claim 27, wherein the diagnostic information types include at least one of:
visual information associated with a lesion candidate which includes at least one of morphological information, intensity information, and segmentation information; and
non-visual information associated with the subject with respect to the liver disease which includes at least one of information extracted from a medical record of the subject and a lab test result associated with the subject.

31. The system according to claim 28, wherein the liver disease types include at least one of Hepatocellular Carcinoma (HCC), Focal Nodular Hyperplasia (FNH), Hemangioma, Cyst, Hepatic Adenoma, and Hepatic Metastasis.

32. The system according to claim 25, wherein a piece of the diagnostic information selectable from the hierarchical representation is embedded with a data manipulation tool with an adjustable operational parameter.

33. The system according to claim 32, wherein the embedded data manipulation tool can be applied to a data set with the adjustable operational parameter to produce adjusted diagnostic information.

34. The system according to claim 33, wherein the system is arranged to update the diagnostic information included in the hierarchical representation using the adjusted diagnostic information.

35. The system according to claim 25, wherein a piece of the diagnostic information selectable from the hierarchical representation is displayed with an alert level estimating a seriousness with respect to the selected lesion candidate computed based on the piece of diagnostic information.

36. The system according to claim 25, wherein the summary with respect to each category of the diagnostic information in the hierarchical representation includes an alert level estimating a seriousness with respect to the selected lesion candidate computed based on the category of the diagnostic information.

37. The system according to claim 25, wherein the overall alert level estimates an overall level of seriousness of the selected lesion candidate based on all categories of information represented in the hierarchical representation.

38. The system according to claim 23, wherein the fourth region further comprises:
a first sub-area configured to record a diagnosis with respect to the selected lesion candidate; and
a second sub-area configured to record a confidence with respect to the diagnosis.

39. The system according to claim 25, further comprising means to activate integration of the selectable diagnostic information in the hierarchical representation to assist reaching a diagnosis with respect to a selected lesion.

40. The system according to claim 1, wherein the visual data manipulation mechanism is further capable of rendering a liver disease reporting page which includes at least one of:
a first portion configured to provide information related to the subject;
a second portion configured to provide non-visual diagnostic information associated with each lesion included in a list of liver lesions;
a third portion configured to provide visual diagnostic information associated with each lesion included in the list of liver lesions; and
a fourth portion configured to provide a diagnosis for each lesion included in the list of liver lesions.

41. The system according to claim 40, wherein the second portion further comprises:
a first sub-portion configured to provide each lesion included in the list of liver lesions and information associated therewith;
a second sub-portion configured to provide a diagnostic summary; and a third sub-portion configured to provide supporting medical evidence related to diagnosis of the list of liver lesions.

42. The system according to claim 41, wherein the information associated with each lesion includes at least one of:
an estimated location of the lesion;
an estimated dimension of the lesion;
an estimated volume of the lesion;
a medical diagnosis of the lesion; and
a measure indicating a confidence in the medical diagnosis.

43. The system according to claim 1, wherein the data manipulation mechanism comprises at least one of:
a data visualization mechanism capable of displaying a data set based on a display parameter;
a data enhancement mechanism capable of being activated to generate an enhanced version of a data set;
a lesion detection mechanism capable of being activated for detecting a lesion candidate in a data set; and
a feature extraction mechanism capable of being activated to extract one or more features associated with a detected lesion candidate,
wherein the enhanced version of a data set is one of a liver intensity subtracted (LIST) image or an interpolated slice image generated by interpolating based on more than one corresponding slice image identified across a time sequence data set.

44. The system according to claim 43, wherein, in the case where the enhanced version of a data set is a LIST image, the enhanced version of a data set is obtained by subtracting pixel values of a first LIST image from corresponding pixel values of a second LIST image for each pair of adjacent LIST images.

45. The system according to claim 43, wherein said detecting a lesion candidate is performed in one of an automatic mode, an interactive mode, and a manual mode.

46. The system according to claim 43, wherein said extracting is performed in one of an automatic mode, an interactive mode, and a manual mode.

47. The system according to claim 1, further comprising a liver disease diagnosis mechanism, which comprises at least one of:
a hierarchical representation construction mechanism configured to generate a hierarchical representation of selectable visual and/or non-visual diagnostic information;
an interactive data exploration mechanism capable of facilitating real time diagnostic evidence exploration; and
an information assessment mechanism capable of supporting real time data rendering to facilitate information assessment.

48. The system according to claim 47, wherein at least some of the diagnostic information in the hierarchical representation is embedded with a data manipulation tool.

49. The system according to claim 1, further comprising a liver disease diagnosis report generation mechanism capable of being activated to produce a liver disease diagnosis report.

50. A method, comprising:
loading one or more liver visual data sets and non-visual information that is associated with a subject and specific to a liver disease;
activating a visual data manipulation page for manipulating the one or more liver visual data sets, wherein the visual data manipulation page includes:
a first area for displaying images of the one or more data sets and for manipulating the one or more data sets, and
a second area for providing a plurality of selectable means to activate one or more data manipulation operations to be performed with respect to the one or more data sets displayed in the first area, wherein
when the first area is configured to manipulate more than one data set, each image is from a corresponding data set and images from different data sets can be displayed synchronously;
visualizing one or more retrieved liver visual data sets in the visual data manipulation page, and
effectuating one or more data manipulation operations, activated via the plurality of selectable means displayed on the visual data manipulation page and to be performed with respect to the one or more data sets, wherein more than one data set can be visualized in a synchronized manner,
wherein a speed of displaying different slices of a data set of volumetric data in the first area is determined based on a distance of a displayed slice from a lesion candidate slice.

51. The method according to claim 50, further comprising selecting one of the selectable means to effectuate a corresponding data manipulation operation, wherein the selected data manipulation operation includes at least one of:
advancing, in a first direction, in a volumetric data set to select a slice to be displayed in the first area;
advancing, in a second direction, in the volumetric data set to select a slice to be displayed in the first area;
adjusting a display parameter associated with a data set displayed in the first area; activating a process of automatic lesion detection applied to a data set displayed in the first area;
activating an interactive process of lesion detection applied to a data set displayed in the first area;
specifying one or more data sets to be displayed in the first area;
manipulating information associated with a list of lesion candidates detected from a data set displayed in the first area; and
deleting information associated with a lesion candidate included in the list of lesion candidates.

52. The method according to claim 50, further comprising activating a liver disease diagnostic page, which enables at least one of:
selecting a lesion candidate from a list of selectable lesion candidates detected from at least one data set;
displaying visual information related to the selected lesion candidate;
exploring at least one piece of diagnostic information associated with the selected lesion candidate and represented by a hierarchy of selectable diagnostic information; and
reaching a diagnosis with respect to the lesion candidate selected based on the at least one piece of diagnostic information.

53. The method according to claim 52, wherein the visual information includes a 3D rendering of the selected lesion candidate.

54. The method according to claim 52, wherein the hierarchy fuses visual and non-visual diagnostic information.

55. The method according to claim 52, wherein at least some of the diagnostic information in the hierarchical representation is embedded with a data manipulation tool.

56. The method according to claim 52, wherein said reaching a diagnosis is performed in one of an automatic mode, an interactive mode, and a combination thereof.

57. The method according to claim 56, wherein said exploring includes:
modifying a piece of diagnostic information to produce an updated piece of diagnostic information; and
assessing a different piece of diagnostic information related to the piece of diagnostic information based on the updated piece of diagnostic information;
evaluating a diagnosis for the selected lesion made based on the piece of diagnostic information using the updated piece of diagnostic information.

58. The method according to claim 57, wherein said modifying is achieved in one of a manual mode, an interactive mode, an automatic mode, and a combination thereof.

59. The method according to claim 57, wherein said modifying is achieved using a data manipulation tool embedded with the piece of diagnostic information.

60. The method according to claim 52, wherein said exploring comprises:
detecting one or more objects imaged in a selected 3D volume visual diagnostic information;
dissecting the 3D volume into a plurality of portions, with at least one portion having at least one of the objects therein; and
pulling, electronically, one of the portions apart from other remaining portions to view a spatial relationship among the objects.

61. The method according to claim 60, wherein the one or more objects include at least one of a liver, a lesion, and a blood vessel.

62. The method according to claim 60, further comprising re-assembling the one pulled apart portion with the other remaining portions.

63. The method according to claim 60, further comprising characterizing the spatial relationship.

64. The method according to claim 63, further comprising deriving a medical decision based on a characterization of the spatial relationship from said characterizing.

65. The method according to claim 64, wherein the medical decision includes at least one of a treatment plan and a surgical plan with respect to the selected lesion.

66. The method according to claim 52, further comprising:
displaying the selected piece of diagnostic information;
presenting a summary of the diagnostic information associated with the lesion candidate selected; and
displaying information related to an overall level of alert with respect to the lesion candidate selected.

67. The method according to claim 52, further comprising activating a means to perform integration of one or more pieces of the selectable diagnostic information from the hierarchical representation to assist reaching a diagnosis with respect to a selected lesion.

68. The method according to claim 50, further comprising generating a liver disease report, which includes at least one of:
information related to the subject;
non-visual diagnostic information associated with each lesion included in a list of liver lesions;
visual diagnostic information associated with each lesion included in the list of liver lesions; and
a diagnosis for each lesion included in the list of liver lesions.

## Patentansprüche

1. Ein System zur Diagnose von Lebererkrankungen, das Folgendes umfasst:
einen Datensammler, der in der Lage ist, einen oder mehrere visuelle Leber-Datensätze und nicht-visuelle Informationen, die mit einem Subjekt (*subject*) assoziiert und für eine Leberkrankheit spezifisch sind, abzurufen; und
ein Mechanismus zur Manipulation von visuellen Daten, der in der Lage ist, Folgendes zu bewerkstelligen:
(i) Rendern einer Seite zur Manipulation von visuellen Daten, wobei die Seite zur Manipulation von visuellen Daten Folgendes umfasst:
einen ersten Bereich zum Anzeigen von Bildern aus einem oder mehreren Datensätzen und zum Manipulieren des einen oder der mehrerer Datensätze und
einen zweiten Bereich zum Bereitstellen einer Vielzahl von wählbaren Mitteln zur Aktivierung einer oder mehrerer Datenmanipulations-Operationen, die im Zusammenhang mit dem einen oder den mehreren im ersten Bereich angezeigten Datensätzen durchgeführt werden sollen,
wobei, wenn der erste Bereich zur Manipulation von mehr als einem Datensatz konfiguriert ist, jedes Bild aus einem entsprechenden Datensatz stammt und Bilder aus verschiedenen Datensätzen synchron angezeigt werden können;
(ii) Visualisieren eines oder mehrerer abgerufener visueller Leber-Datensätze auf der Seite zur Manipulation von visuellen Daten, und
(iii) Ausführen einer oder mehrerer Datenmanipulations-Operationen, die über die Vielzahl von wählbaren Mitteln aktiviert werden, die auf der Seite zur Manipulation von visuellen Daten angezeigt werden und in Bezug auf den einen oder die mehreren Datensätze ausgeführt werden sollen, wobei mehr als ein Datensatz auf synchronisierte Weise visualisiert werden kann,
wobei eine Geschwindigkeit bei der Anzeige verschiedener Schnitte (*slices*) eines Datensatzes von volumetrischen Daten im ersten Bereich bestimmt wird auf der Grundlage eines Abstands eines angezeigten Schnitts von einem Läsionskandidaten-Schnitt.

2. Das System nach Anspruch 1, wobei ein visueller Datensatz zur Leber ein Bild und/oder ein Volumen enthält, das in einer Bildgebungsmodalität erfasst wurde.

3. Das System nach Anspruch 2, wobei die Bildgebungsmodalität mindestens eine der Folgenden umfasst: Computertomografie (CT), Magnetresonanztomografie (MRI) und Positronenemissionstomografie (PET).

4. Das System nach Anspruch 3, wobei die CT-Leberdaten Daten verschiedener Phasen umfassen, einschließlich mindestens einer der folgenden Phasen: eine einfache CT-Phase, eine arterielle Phase, eine venöse Phase und eine verzögerte Phase.

5. Das System nach Anspruch 4, wobei das mehr als eine Bild, die im ersten Bereich angezeigt werden, Bilder aus verschiedenen CT-Phasen sind.

6. Das System nach Anspruch 1, wobei die nicht-visuellen Informationen die in einer Krankenakte des Patienten enthaltenen Informationen beinhalten.

7. Das System nach Anspruch 1, wobei die nicht-visuellen Informationen ein mit der Testperson assoziiertes Labortestergebnis enthalten.

8. Das System nach Anspruch 1, wobei die nicht-visuellen Informationen spezifisch und relevant für den Typ der Leberkrankheit sind.

9. Das System nach Anspruch 1, wobei die Mehrzahl der wählbaren Mittel mindestens eines der folgenden umfasst:
ein anklickbares Symbol, das konfiguriert ist, um in einem volumetrischen Datensatz in einer ersten Richtung vorzurücken, um einen Schnitt auszuwählen, der im ersten Bereich angezeigt werden soll;
ein anklickbares Symbol, das konfiguriert ist, um in einer zweiten Richtung im volumetrischen Datensatz vorzurücken, um einen Schnitt auszuwählen, der im ersten Bereich angezeigt werden soll;
ein anklickbares Symbol, das konfiguriert ist, um einen Anzeigeparameter einzustellen, der mit einem Datensatz assoziiert ist, der im ersten Bereich angezeigt wird;
ein anklickbares Symbol, das konfiguriert ist, um einen Prozess der automatischen Läsionserkennung zu aktivieren, der auf einen im ersten Bereich angezeigten Datensatz angewendet wird;
ein anklickbares Symbol, das konfiguriert ist, um einen interaktiven Prozess der Läsionserkennung zu aktivieren, der auf einen im ersten Bereich angezeigten Datensatz angewendet wird;
ein Unterbereich, der konfiguriert ist, um eine Angabe eines oder mehrerer Datensätze zu erleichtern, die im ersten Bereich angezeigt werden sollen;
einen Unterbereich, der konfiguriert ist, um Informationen zu manipulieren, die mit einer Liste von Läsionskandidaten assoziiert sind, die ausgehend von einem Datensatz erkannt wurden, der im ersten Bereich angezeigt wird; und
ein anklickbares Symbol, das konfiguriert ist, um Informationen zu löschen, die mit einem Läsionskandidaten assoziiert sind, der in der Liste der Läsionskandidaten enthalten ist.

10. Das System nach Anspruch 9, wobei ein anklickbares Symbol eine Schaltfläche bzw. ein Taster, bzw. ein Knopf (*button*) ist.

11. Das System nach Anspruch 9, wobei der Unterbereich zur Angabe eines anzuzeigenden Datensatzes Folgendes umfasst:
ein Fenster zur Eingabe einer wählbaren Zahl, die die Anzahl der im ersten Bereich anzuzeigenden Datensätze angibt; und
ein oder mehrere Bereiche, die jeweils einem Unterbereich im ersten Bereich entsprechen, in dem ein Datensatz angezeigt werden soll, die die Angabe eines Datensatzes erleichtern, der im entsprechenden Unterbereich im ersten Bereich manipuliert werden soll.

12. Das System nach Anspruch 11, das ferner Folgendes umfasst: wenn mehr als ein Datensatz angezeigt werden soll, ein Symbol, durch das ein Anzeigemodus über verschiedene Datensätze hinweg definiert wird.

13. Das System nach Anspruch 12, wobei der Anzeigemodus über verschiedene Datensätze hinweg einen der folgenden Modi umfasst: einen synchronen und einen asynchronen Modus.

14. Das System nach Anspruch 9, wobei der Unterbereich zur Manipulation von Informationen, die mit einer Liste von Läsionskandidaten assoziiert sind, Folgendes umfasst:
einen Fensterbereich, in dem die mit einer Liste von Läsionskandidaten assoziierte Informationen angezeigt werden;
ein anklickbares Symbol, das konfiguriert ist, um die Steuerung der Überlagerung von Informationen zu erleichtern, die mit der Liste der Läsionskandidaten im ersten Bereich assoziiert sind, wo der zugrundeliegende Datensatz visualisiert wird.

15. Das System nach Anspruch 9, wobei eine Anpassung des Anzeigeparameters durch eine Mausbewegung / einen Mausklick bewirkt werden kann, wenn das anklickbare Symbol zur Anpassung des Anzeigeparameters angeklickt wird.

16. Das System nach Anspruch 9, wobei die im ersten Bereich anzuzeigende visuelle Information eine erweiterte bzw. angereicherte Version eines Datensatzes ist und wobei die erweiterte bzw. angereicherte Version eines Datensatzes ein oder mehrere Bilder mit subtrahierter Leberintensität (*liver intensity subtracted* - LIST) enthält.

17. Das System nach Anspruch 16, wobei die erweiterte bzw. angereicherte Version eines Datensatzes ein oder mehrere Bilder enthält, die durch Subtraktion benachbarter LIST-Bilder abgeleitet wurden.

18. Das System nach Anspruch 9, wobei die im ersten Bereich anzuzeigende visuelle Information eine erweiterte bzw. angereicherte Version eines Datensatzes ist, und wobei die erweiterte bzw. angereicherte Version eines Datensatzes erhalten wird durch Interpolation von mehr als einem registrierten Schnitt von mehr als einem Datensatz, die zu verschiedenen Zeiten erfasst wurden, und wobei der interpolierte Schnitt als ein animierter Film angezeigt wird.

19. Das System nach Anspruch 9, wobei das System so eingerichtet ist, dass es die automatische Läsionserkennung als *Backend*-Prozess durchführt.

20. Das System nach Anspruch 9, wobei das System so eingerichtet ist, dass es die interaktive Läsionserkennung in Bezug auf eine Markierung durchführt, die erzeugt wird auf der Grundlage einer Visualisierung eines Datensatzes im ersten Bereich.

21. Das System nach Anspruch 20, wobei das System so eingerichtet ist, dass es die automatischen und interaktiven Läsionserkennungs-Prozesse durchführt, um ein oder mehrere Merkmale zu extrahieren, die mit einem Erkennungsergebnis assoziiert sind.

22. Das System nach Anspruch 21, wobei das eine oder die mehreren Merkmale mindestens eines der folgenden Merkmale umfassen:
einen Wahrscheinlichkeitswert, der angibt, wie wahrscheinlich es ist, dass eine Läsion in der Nähe der Markierung vorhanden ist; und
mindestens eine Messung, die durchgeführt wird in Bezug auf jede entdeckte Läsion.

23. Das System nach Anspruch 1, wobei der Mechanismus zur Manipulation von visuellen Daten ferner in der Lage ist, eine diagnostische Seite der Leberkrankheit zu rendern, die mindestens eine der folgenden Komponenten enthält:
eine erste Region, die konfiguriert ist zur Anzeige einer Liste von auswählbaren Läsionskandidaten, die ausgehend von mindestens einem Datensatz erfasst wurden;
eine zweite Region, die konfiguriert ist zum Anzeigen visueller Informationen, die sich auf einen Läsionskandidaten beziehen, die aus der Liste auswählbarer Läsionskandidaten ausgewählt wurden;
eine dritte Region, die zur Anzeige von diagnostischen Informationen konfiguriert ist, die mit dem ausgewählten Läsionskandidaten assoziiert sind; und
eine vierte Region, die konfiguriert ist zur Aufzeichnung von Informationen die sich auf eine Diagnose in Bezug auf den ausgewählten Läsionskandidaten beziehen.

24. Das System nach Anspruch 23, wobei die zweite Region eine oder mehrere Unter-Regionen umfasst, in denen jeweils ein Datensatz oder der ausgewählte Läsionskandidat visualisiert und/oder manipuliert werden kann.

25. Das System nach Anspruch 23, wobei die dritte Region ferner Folgendes umfasst:
eine erste Unterregion, die konfiguriert ist zur Anzeige einer hierarchischen Darstellung von wählbaren diagnostischen Informationen, die sich auf den ausgewählten Läsionskandidaten beziehen;
eine zweite Unterregion, der konfiguriert ist zur Anzeige eines Typs von diagnostischer Information, die ausgewählt wird aus der hierarchischen Darstellung mit Bezug auf den ausgewählten Läsionskandidaten;
eine dritte Unterregion, die konfiguriert ist zur Darstellung einer Zusammenfassung der diagnostischen Information in der hierarchischen Darstellung, die mit dem ausgewählten Läsionskandidaten assoziiert ist; und
eine vierte Unterregion, die konfiguriert ist zur Anzeige von Informationen zu einem Gesamt-Alarmpegel in Bezug auf den ausgewählten Läsionskandidaten.

26. Das System nach Anspruch 25, wobei die hierarchische Darstellung visuelle und/oder nicht-visuelle diagnostische Informationen enthält.

27. Das System nach Anspruch 25, wobei die hierarchische Darstellung Informationen auf der Grundlage von diagnostischen Informationstypen organisiert.

28. Das System nach Anspruch 25, wobei die hierarchische Darstellung Informationen auf der Grundlage von Leberkrankheits-Typen organisiert.

29. Das System nach Anspruch 25, wobei jede Informationseinheit aus den auswählbaren diagnostischen Informationen in der hierarchischen Darstellung durch einen Mausklick ausgewählt werden kann.

30. Das System nach Anspruch 27, wobei die diagnostischen Informationstypen mindestens einen der folgenden Typen umfassen:
visuelle Informationen, die mit einem Läsionskandidaten assoziiert sind, die mindestens eine der folgenden Informationen enthalten: morphologische Informationen, Intensitätsinformationen und Segmentierungsinformationen; und
nicht-visuelle Informationen, die mit der Person bezüglich der Leberkrankheit assoziiert sind, die mindestens einer der Informationen beinhalten, die aus einer medizinischen Akte der Person und einem mit der Person assoziierten Labortestergebnis gewonnen wurden.

31. Das System nach Anspruch 28, wobei die Leberkrankheits-Typen mindestens eines der folgenden umfassen: ein Hepatozelluläres Karzinom (HCC), eine fokale noduläre Hyperplasie (FNH), ein Hämangiom, eine Zyste, ein hepatisches Adenom und eine Lebermetastase.

32. Das System nach Anspruch 25, wobei ein Teil der diagnostischen Informationen, der aus der hierarchischen Darstellung auswählbar ist, mit einem Datenmanipulationswerkzeug mit einem einstellbaren Betriebsparameter eingebettet ist.

33. Das System nach Anspruch 32, wobei das eingebettete Datenmanipulationswerkzeug auf einen Datensatz mit dem einstellbaren Betriebsparameter angewendet werden kann, um angepasste diagnostische Informationen zu erzeugen.

34. Das System nach Anspruch 33, wobei das System so ausgelegt ist, dass es die in der hierarchischen Darstellung enthaltenen diagnostischen Informationen unter Verwendung der angepassten diagnostischen Informationen aktualisiert.

35. Das System nach Anspruch 25, wobei ein Teil der aus der hierarchischen Darstellung wählbaren diagnostischen Informationen mit einem Alarmpegel angezeigt wird, der eine Ernsthaftigkeit bzw. Schwere bezüglich des ausgewählten Läsionskandidaten abschätzt, der auf der Grundlage des Teils der diagnostischen Information berechnet wird.

36. Das System nach Anspruch 25, wobei die Zusammenfassung bezüglich jeder Kategorie der diagnostischen Information in der hierarchischen Darstellung einen Alarmpegel enthält, der eine Schwere bezüglich der ausgewählten Läsionskandidaten schätzt, der auf der Grundlage der Kategorie der diagnostischen Information berechnet wird.

37. Das System nach Anspruch 25, wobei der Gesamt-Alarmpegel einen Gesamt-Schweregrad des ausgewählten Läsionskandidaten auf der Grundlage aller Informations-Kategorien schätzt, die in der hierarchischen Darstellung dargestellt sind.

38. Das System nach Anspruch 23, wobei die vierte Region ferner Folgendes umfasst:
einen ersten Unterbereich, der konfiguriert ist, um eine Diagnose bezüglich des ausgewählten Läsionskandidaten aufzuzeichnen; und
einen zweiten Unterbereich, der konfiguriert ist, um ein Vertrauen (*confidence*) bezüglich der Diagnose aufzuzeichnen.

39. Das System nach Anspruch 25, das ferner Mittel umfasst zur Aktivierung der Integration der wählbaren diagnostischen Informationen in die hierarchische Darstellung, um das Erreichen einer Diagnose bezüglich einer ausgewählten Läsion zu unterstützen.

40. Das System nach Anspruch 1, wobei der Mechanismus zur Manipulation von visuellen Daten ferner in der Lage ist, eine Berichterstellungs-Seite (*reporting page*) über eine Lebererkrankung zu rendern, die mindestens eine der folgenden Informationen enthält:
einen ersten Abschnitt, der so konfiguriert ist, dass er Informationen über die Person liefert;
einen zweiten Abschnitt, der so konfiguriert ist, dass er nicht-visuelle diagnostische Informationen liefert, die mit jeder Läsion in einer Liste von Leberläsionen assoziiert sind;
einen dritten Abschnitt, der so konfiguriert ist, dass er visuelle diagnostische Informationen liefert, die mit jeder Läsion in einer Liste von Leberläsionen assoziiert sind; und
einen vierten Abschnitt, der konfiguriert ist, um eine Diagnose für jede Läsion zu liefern, die in der Liste der Leberläsionen enthalten ist.

41. Das System nach Anspruch 40, wobei der zweite Abschnitt ferner Folgendes umfasst:
einen ersten Unterabschnitt, der konfiguriert ist, um jede in der Liste der Leberläsionen enthaltene Läsion und die damit verbundenen Informationen bereitzustellen;
einen zweiten Unterabschnitt, der konfiguriert ist, um eine diagnostische Zusammenfassung zu liefern; und
einen dritten Unterabschnitt, der konfiguriert ist, um unterstützende medizinische Befunde bezüglich der Diagnose der Liste der Leberläsionen bereitzustellen.

42. Das System nach Anspruch 41, wobei die mit jeder Läsion assoziierten Informationen mindestens eine der folgenden Angaben enthalten:
eine geschätzte Lage der Läsion;
eine geschätzte Abmessung der Läsion;
ein geschätztes Volumen der Läsion;
eine medizinische Diagnose der Läsion; und
ein Maß für das Vertrauen in die medizinische Diagnose.

43. Das System nach Anspruch 1, wobei der Datenmanipulations-Mechanismus mindestens einen der Folgenden umfasst:
einen Datenvisualisierungs-Mechanismus, der in der Lage ist, einen Datensatz anzuzeigen, der auf einem Anzeigeparameter basiert;
einen Datenanreicherungs-Mechanismus, der aktiviert werden kann, um eine angereicherte Version eines Datensatzes zu erzeugen;
einem Läsionserkennungs-Mechanismus, der zum Erkennen eines Läsionskandidaten in einem Datensatz aktiviert werden kann; und
ein Merkmalsextraktion-Mechanismus, der aktiviert werden kann, um ein oder mehrere Merkmale zu extrahieren, die mit einem erkannten Läsionskandidaten assoziiert sind,
wobei die angereicherte Version eines Datensatzes eine der Folgenden ist: ein Bild mit subtrahierter Leberintensität (LIST) oder ein interpoliertes Schnittbild ist, das durch Interpolation erzeugt wird auf der Grundlage von mehr als einem entsprechenden Schnittbild, das über einen Zeitsequenz-Datensatz identifiziert wurde.

44. Das System nach Anspruch 43, wobei im Fall, dass die angereicherte Version eines Datensatzes ein LIST-Bild ist, die angereicherte Version eines Datensatzes erhalten wird durch Subtraktion von Pixelwerten eines ersten LIST-Bildes von entsprechenden Pixelwerten eines zweiten LIST-Bildes für jedes Paar benachbarter LIST-Bilder.

45. Das System nach Anspruch 43, wobei das genannte Erkennen eines Läsionskandidaten in einem der folgenden Modi durchgeführt wird: in einem automatischen Modus, in einem interaktiven Modus oder in einem manuellen Modus.

46. Das System nach Anspruch 43, wobei das genannte Extrahieren in einem der folgenden Modi durchgeführt wird: in einem automatischen Modus, in einem interaktiven Modus oder in einem manuellen Modus.

47. Das System nach Anspruch 1, das ferner einen Mechanismus zur Diagnose von Lebererkrankungen umfasst, der mindestens eines der folgenden Elemente umfasst:
einen Mechanismus zum Aufbau einer hierarchischen Darstellung, der konfiguriert ist, um eine hierarchische Darstellung von wählbaren visuellen und/oder nicht-visuellen diagnostischen Informationen zu erzeugen;
einem interaktiven Datenexplorations-Mechanismus, der in der Lage ist, eine Echtzeit-Exploration diagnostischer Befunde zu erleichtern; und
ein Mechanismus zur Informationsbeurteilung (*assessment*)*,* der in der Lage ist, ein Echtzeit-Daten-*Rendering* zu unterstützen, um die Beurteilung der Informationen zu erleichtern.

48. Das System nach Anspruch 47, wobei zumindest ein Teil der diagnostischen Informationen in der hierarchischen Darstellung mit einem Datenmanipulationswerkzeug eingebettet ist.

49. Das System nach Anspruch 1, das ferner einen Mechanismus zur Erzeugung eines Leberkrankheits-Diagnoseberichts umfasst, der aktiviert werden kann, um einen Leberkrankheits-Diagnosebericht zu erstellen.

50. Ein Verfahren, das Folgendes umfasst:
Laden eines oder mehrerer visueller Leber-Datensätze und nicht-visueller Informationen, die einem Subjekt zugehörig und für eine Leberkrankheit spezifisch sind;
Aktivieren einer Seite zur Manipulation visueller Daten zum Manipulieren des einen oder der mehreren visuellen Leber-Datensätze, wobei die Seite zur Manipulation visueller Daten Folgendes enthält:
einen ersten Bereich zum Anzeigen von Bildern aus einem oder mehreren Datensätze und zum Manipulieren des einen oder der mehrerer Datensätze und
einen zweiten Bereich zum Bereitstellen einer Vielzahl von wählbaren Mitteln zur Aktivierung einer oder mehrerer Datenmanipulations-Operationen, die im Zusammenhang mit dem einen oder den mehreren im ersten Bereich angezeigten Datensätzen durchgeführt werden sollen, wobei
wenn der erste Bereich zur Manipulation von mehr als einem Datensatz konfiguriert ist, jedes Bild aus einem entsprechenden Datensatz stammt und Bilder aus verschiedenen Datensätzen synchron angezeigt werden können;
Visualisieren eines oder mehrerer abgerufener visueller Leber-Datensätze auf der Seite zur Manipulation visueller Daten, und
Ausführen einer oder mehrerer Datenmanipulations-Operationen, die über die Vielzahl von wählbaren Mitteln aktiviert werden, die auf der Seite zur Manipulation von visuellen Daten angezeigt werden und in Bezug auf den einen oder die mehreren Datensätze ausgeführt werden sollen, wobei mehr als ein Datensatz auf synchronisierte Weise visualisiert werden kann,
wobei eine Geschwindigkeit bei der Anzeige verschiedener Schnitte (*slices*) eines Datensatzes von volumetrischen Daten im ersten Bereich bestimmt wird auf der Grundlage eines Abstands eines angezeigten Schnitts von einem Läsionskandidaten-Schnitt.

51. Das Verfahren nach Anspruch 50, das ferner das Auswählen eines der auswählbaren Mittel zur Durchführung einer entsprechenden Datenmanipulations-Operation umfasst, wobei die ausgewählte Datenmanipulations-Operation mindestens eine der folgenden Operationen umfasst:
Vorwärtsbewegen, in einer ersten Richtung, in einem volumetrischen Datensatz, um einen Schnitt auszuwählen, der im ersten Bereich angezeigt werden soll;
Vorwärtsbewegen, in einer zweiten Richtung, im volumetrischen Datensatz, um einen Schnitt auszuwählen, die im ersten Bereich angezeigt werden soll;
Einstellen eines Anzeigeparameters, der mit einem im ersten Bereich angezeigten Datensatz assoziiert ist; Aktivieren eines Prozesses der automatischen Läsionserkennung, der auf einen im ersten Bereich angezeigten Datensatz angewendet wird;
Aktivieren eines interaktiven Prozesses der Läsionserkennung, der auf einen im ersten Bereich angezeigten Datensatz angewendet wird;
Spezifizieren eines oder mehrerer Datensätze, die im ersten Bereich angezeigt werden sollen;
Manipulieren von Informationen, die mit einer Liste von Läsionskandidaten assoziiert sind, die aus einem im ersten Bereich angezeigten Datensatz entdeckt wurden; und
Löschen von Informationen, die mit einem Läsionskandidaten assoziiert sind, der in der Liste der Läsionskandidaten enthalten ist.

52. Das Verfahren nach Anspruch 50, das ferner das Aktivieren einer Diagnoseseite für Lebererkrankungen umfasst, die mindestens eine der folgenden Möglichkeiten bietet:
Auswählen eines Läsionskandidaten aus einer Liste von auswählbaren Läsionskandidaten, die aus mindestens einem Datensatz entdeckt wurden;
Anzeigen visueller Informationen, die sich auf den ausgewählten Läsionskandidaten beziehen;
Erkunden von mindestens einer diagnostischen Information, die mit dem ausgewählten Läsionskandidaten assoziiert ist und durch eine Hierarchie von auswählbaren diagnostischen Informationen dargestellt wird; und
Erreichen einer Diagnose bezüglich des ausgewählten Läsionskandidaten auf der Grundlage von mindestens einer diagnostischen Informationseinheit.

53. Das Verfahren nach Anspruch 52, wobei die visuelle Information ein 3D*-Rendering* des ausgewählten Läsionskandidaten beinhaltet.

54. Das Verfahren nach Anspruch 52, wobei die Hierarchie visuelle und nicht-visuelle diagnostische Informationen zusammenführt bzw. verschmilzt.

55. Das Verfahren nach Anspruch 52, wobei zumindest ein Teil der diagnostischen Informationen in der hierarchischen Darstellung mit einem Datenmanipulationswerkzeug eingebettet wird.

56. Das Verfahren nach Anspruch 52, wobei das genannte Erreichen einer Diagnose in einem der folgenden Modi durchgeführt wird: in einem automatischen Modus, in einem interaktiven Modus und in einer Kombination davon.

57. Das Verfahren nach Anspruch 56, wobei das genannte Erkunden Folgendes umfasst:
Modifizieren einer diagnostischen Informationseinheit, um eine aktualisierte diagnostische Informationseinheit zu erzeugen; und
Beurteilen einer anderen diagnostischen Informationseinheit, die mit der diagnostischen Informationseinheit zusammenhängt, basierend auf der aktualisierten diagnostischen Informationseinheit;
Auswerten einer Diagnose für die ausgewählte Läsion, die auf der Grundlage der diagnostischen Informationseinheit erstellt wurde unter Verwendung der aktualisierten diagnostischen Informationseinheit.

58. Das Verfahren nach Anspruch 57, wobei das genannte Modifizieren in einem der folgenden Modi erreicht wird: in einem manuellen Modus, in einem interaktiven Modus, in einem automatischen Modus, und in einer Kombination davon.

59. Das Verfahren nach Anspruch 57, wobei das genannte Modifizieren erreicht wird unter Verwendung eines Datenmanipulationswerkzeugs, das mit den diagnostischen Informationen eingebettet ist.

60. Das Verfahren nach Anspruch 52, wobei das genannte Erkunden Folgendes umfasst:
Erfassen eines oder mehrerer Objekte, die in einer ausgewählten visuellen diagnostischen Information als 3D-Volumen abgebildet sind;
Zerlegen des 3D-Volumens in eine Vielzahl von Abschnitten, wobei mindestens ein Abschnitt mindestens eines der Objekte darin aufweist; und
Auseinanderziehen, auf elektronische Weise, von einem der Abschnitte von den anderen verbleibenden Abschnitten, um eine räumliche Beziehung zwischen den Objekten zu erkennen.

61. Das Verfahren nach Anspruch 60, wobei das eine oder die mehreren Objekte mindestens eines der Folgenden beinhalten: eine Leber, eine Läsion und ein Blutgefäß.

62. Das Verfahren nach Anspruch 60, das ferner Folgendes umfasst: das Wiederzusammensetzen des einen auseinandergezogenen Abschnitts mit den anderen verbleibenden Abschnitten.

63. Das Verfahren nach Anspruch 60, das ferner das Charakterisieren der räumlichen Beziehung umfasst.

64. Das Verfahren nach Anspruch 63, das ferner das Ableiten einer medizinischen Entscheidung auf der Grundlage einer Charakterisierung der räumlichen Beziehung umfasst, ausgehend vom genannten Charakterisieren.

65. Das Verfahren nach Anspruch 64, wobei die medizinische Entscheidung mindestens einen der Folgenden umfasst: einen Behandlungsplan und/oder einen chirurgischen Plan bezüglich der ausgewählten Läsion.

66. Das Verfahren nach Anspruch 52, das ferner Folgendes umfasst:
Anzeigen der ausgewählten diagnostischen Informationseinheit;
Präsentieren einer Zusammenfassung der diagnostischen Informationen, die mit dem ausgewählten Läsionskandidaten assoziiert sind; und
Anzeigen von Informationen bezüglich eines allgemeinen Alarmniveaus in Zusammenhang mit dem ausgewählten Läsionskandidaten.

67. Das Verfahren nach Anspruch 52, das ferner das Aktivieren eines Mittels zur Durchführung der Integration eines oder mehrerer Einheiten der auswählbaren diagnostischen Informationen aus der hierarchischen Darstellung umfasst, um das Erreichen einer Diagnose bezüglich einer ausgewählten Läsion zu unterstützen.

68. Das Verfahren nach Anspruch 50, das ferner das Erzeugen eines Lebererkrankungsberichts umfasst, der mindestens eines der folgenden Elemente enthält:
Informationen, die sich auf das Subjekt beziehen;
nicht-visuelle diagnostische Informationen, die mit jeder Läsion assoziiert sind, die in einer Liste von Leberläsionen enthalten ist;
visuelle diagnostische Informationen, die mit jeder Läsion assoziiert sind, die in einer Liste von Leberläsionen enthalten ist; und
eine Diagnose für jede in der Liste der Leberläsionen enthaltene Läsion.

## Revendications

1. Un système de diagnostic des maladies du foie, comprenant :
un récupérateur de données capable de récupérer un ou plusieurs ensembles de données visuelles sur le foie et des informations non visuelles associées à un sujet et spécifiques à une maladie du foie ; et
un mécanisme de manipulation de données visuelles capable de :
(i) restituer une page de manipulation de données visuelles, sachant que la page de manipulation de données visuelles inclut :
une première zone permettant d'afficher des images d'un ou plusieurs ensembles de données et pour manipuler le ou les ensembles de données, et
une deuxième zone permettant de fournir une pluralité de moyens sélectionnables pour activer une ou plusieurs opérations de manipulation de données à effectuer en ce qui concerne le ou les ensembles de données affichés dans la première zone,
sachant que, lorsque la première zone est configurée pour manipuler plus d'un ensemble de données, chaque image provient d'un ensemble de données correspondant et des images de différents ensembles de données peuvent être affichées de manière synchrone ;
(ii) visualiser dans la page de manipulation des données visuelles un ou plusieurs ensembles de données visuelles du foie récupérées, et
(iii) effectuer une ou plusieurs opérations de manipulation de données, activées via la pluralité de moyens sélectionnables affichés sur la page de manipulation de données visuelles, et devant être effectuées par rapport à un ou plusieurs ensembles de données, sachant que plus d'un ensemble de données peut être visualisé de manière synchronisée,
sachant qu'une vitesse d'affichage de différentes tranches d'un ensemble de données volumétriques dans la première zone est déterminée sur la base d'une distance entre une tranche affichée et une tranche candidate à la lésion.

2. Le système d'après la revendication 1, sachant qu'un ensemble de données visuelles du foie inclut une image et/ou un volume acquis dans une modalité d'imagerie.

3. Le système d'après la revendication 2, sachant que la modalité d'imagerie inclut au moins l'une des techniques suivantes : la tomographie informatisée (CT), l'imagerie par résonance magnétique (IRM) et la tomographie par émission de positrons (TEP).

4. Le système d'après la revendication 3, sachant que les données de tomographie informatisée du foie comprennent des données de différentes phases, dont au moins une d'une phase de tomographie informatisée simple, d'une phase artérielle, d'une phase veineuse et d'une phase retardée.

5. Le système d'après la revendication 4, sachant que les plus d'une image qui sont affichées dans la première zone sont des images provenant de différentes phases de CT.

6. Le système d'après la revendication 1, sachant que les informations non visuelles incluent des informations contenues dans un dossier médical du sujet.

7. Le système d'après la revendication 1, sachant que les informations non visuelles incluent un résultat de test de laboratoire associé au sujet.

8. Le système d'après la revendication 1, sachant que les informations non visuelles sont spécifiques et pertinentes pour le type de maladie du foie.

9. Le système d'après la revendication 1, sachant que la pluralité de moyens sélectionnables inclut au moins un des éléments suivants :
une icône cliquable configurée pour avancer, dans une première direction, dans un ensemble de données volumétriques afin de sélectionner une tranche à afficher dans la première zone ;
une icône cliquable configurée pour avancer, dans une deuxième direction, dans l'ensemble de données volumétriques afin de sélectionner une tranche à afficher dans la première zone ;
une icône cliquable configurée pour ajuster un paramètre d'affichage associé à un ensemble de données affiché dans la première zone ;
une icône cliquable configurée pour activer un processus de détection automatique des lésions appliqué à un ensemble de données affiché dans la première zone ;
une icône cliquable configurée pour activer un processus interactif de détection de lésions appliqué à un ensemble de données affiché dans la première zone ;
une sous-zone configurée pour faciliter la spécification d'un ou plusieurs ensembles de données à afficher dans la première zone ;
une sous-zone configurée pour manipuler des informations associées à une liste de lésions candidates détectées à partir d'un ensemble de données affiché dans la première zone ; et
une icône cliquable configurée pour supprimer des informations associées à une lésion candidate comprise dans la liste des lésions candidates.

10. Le système d'après la revendication 9, sachant qu'une icône cliquable est un bouton ou encore une touche (*button*)*.*

11. Le système d'après la revendication 9, sachant que la sous-zone pour la spécification d'un ensemble de données à afficher comprend :
une fenêtre permettant d'entrer un numéro sélectionnable indiquant le nombre d'ensembles de données à afficher dans la première zone ; et
une ou plusieurs zones, chacune correspondant à une sous-zone de la première zone dans laquelle un ensemble de données doit être affiché, facilitant la spécification d'un ensemble de données à manipuler dans la sous-zone correspondante dans la première zone.

12. Le système d'après la revendication 11, comprenant en outre, lorsque plus d'un ensemble de données doit être affiché, une icône par laquelle est défini un mode d'affichage à travers différents ensembles de données.

13. Le système d'après la revendication 12, sachant que le mode d'affichage à travers différents ensembles de données inclut un mode synchronisé et un mode asynchronisé.

14. Le système d'après la revendication 9, sachant que la sous-zone pour manipuler des informations associées à une liste de lésions candidates comprend :
une zone de fenêtre dans laquelle sont affichées les informations associées à une liste de lésions candidates ;
une icône cliquable configurée pour faciliter le contrôle de la superposition des informations associées à la liste de lésions candidates dans la première zone où l'ensemble des données sous-jacentes est visualisé.

15. Le système d'après la revendication 9, sachant qu'un ajustement du paramètre d'affichage peut être effectué par un mouvement/clic de la souris lorsque l'icône cliquable pour l'ajustement du paramètre d'affichage est cliquée.

16. Le système d'après la revendication 9, sachant que des informations visuelles à afficher dans la première zone sont une version enrichie ou encore améliorée d'un ensemble de données, et sachant que la version améliorée d'un ensemble de données inclut une ou plusieurs images soustraites de l'intensité du foie (LIST).

17. Le système d'après la revendication 16, sachant que la version améliorée d'un ensemble de données inclut une ou plusieurs images obtenues par soustraction d'images LIST adjacentes.

18. Le système d'après la revendication 9, sachant que des informations visuelles à afficher dans la première zone sont une version améliorée d'un ensemble de données, et sachant que la version améliorée d'un ensemble de données est obtenue en interpolant plus d'une tranche enregistrée de plus d'un ensemble de données correspondant acquis à différents moments, et que la tranche interpolée est affichée comme un film d'animation.

19. Le système d'après la revendication 9, sachant que le système est agencé pour effectuer la détection automatique de lésions en tant que processus d'arrière-plan (*backend*)*.*

20. Le système d'après la revendication 9, sachant que le système est agencé pour effectuer la détection interactive de lésions par rapport à un marquage créé sur la base d'une visualisation d'un ensemble de données dans la première zone.

21. Le système d'après la revendication 20, sachant que le système est agencé pour effectuer les processus de détection de lésions automatique et interactif afin d'extraire une ou plusieurs caractéristiques associées à un résultat de détection.

22. Le système d'après la revendication 21, sachant que la ou les caractéristiques incluent au moins l'une des suivantes :
une valeur de vraisemblance indiquant à quel point il est probable qu'une lésion soit présente à proximité du marquage ; et
au moins une mesure effectuée en rapport avec chaque lésion détectée.

23. Le système d'après la revendication 1, sachant que le mécanisme de manipulation de données visuelles est en outre capable de produire un rendu d'une page de diagnostic de maladie du foie, qui comprend au moins un des éléments suivants :
une première région configurée pour afficher une liste de lésions candidates sélectionnables détectées à partir d'au moins un ensemble de données ;
une deuxième région configurée pour afficher des informations visuelles relatives à une lésion candidate sélectionnée dans la liste des lésions candidates sélectionnables ;
une troisième région configurée pour afficher des informations de diagnostic associées à la lésion candidate sélectionnée ; et
une quatrième région configurée pour enregistrer des informations relatives à un diagnostic en rapport avec la lésion candidate sélectionnée.

24. Le système d'après la revendication 23, sachant que la deuxième région comprend une ou plusieurs sous-régions, dans chacune desquelles un ensemble de données ou la lésion candidate sélectionnée peut être visualisé et/ou manipulé.

25. Le système d'après la revendication 23, sachant que la troisième région comprend en outre :
une première sous-région configurée pour afficher une représentation hiérarchique des informations de diagnostic sélectionnables relatives à la lésion candidate sélectionnée ;
une deuxième sous-région configurée pour afficher un type d'informations de diagnostic sélectionnées à partir de la représentation hiérarchique en rapport avec la lésion candidate sélectionnée ;
une troisième sous-région configurée pour présenter un résumé des informations de diagnostic dans la représentation hiérarchique qui est associé à la lésion candidate sélectionnée ; et
une quatrième sous-région configurée pour afficher des informations relatives à un niveau d'alerte général en rapport avec la lésion candidate sélectionnée.

26. Le système d'après la revendication 25, sachant que la représentation hiérarchique inclut des informations de diagnostic visuelles et/ou non visuelles.

27. Le système d'après la revendication 25, sachant que la représentation hiérarchique organise des informations en fonction des types d'informations de diagnostic.

28. Le système d'après la revendication 25, sachant que la représentation hiérarchique organise des information en fonction des types de maladies du foie.

29. Le système d'après la revendication 25, sachant que chaque élément des informations de diagnostic sélectionnables dans la représentation hiérarchique peut être sélectionné par un clic de souris.

30. Le système d'après la revendication 27, sachant que les types d'informations de diagnostic incluent au moins un des éléments suivants :
des informations visuelles, associées à une lésion candidate, qui incluent au moins l'une parmi les informations suivantes : des informations morphologiques, des informations d'intensité et des informations de segmentation ; et
des informations non visuelles, associées au sujet en ce qui concerne la maladie du foie, qui incluent au moins l'un parmi les suivants : des informations extraites d'un dossier médical du sujet et un résultat de test de laboratoire associé au sujet.

31. Le système d'après la revendication 28, sachant que les types de maladies du foie incluent au moins l'un des types suivants : un carcinome hépatocellulaire (HCC), une hyperplasie nodulaire focale (FNH), un hémangiome, une kyste, un adénome hépatique et une métastase hépatique.

32. Le système d'après la revendication 25, sachant qu'un élément des informations de diagnostic sélectionnable à partir de la représentation hiérarchique est intégré à un outil de manipulation de données avec un paramètre opérationnel réglable.

33. Le système d'après la revendication 32, sachant que l'outil de manipulation de données intégré peut être appliqué à un ensemble de données avec le paramètre opérationnel ajustable pour produire des informations de diagnostic ajustées.

34. Le système d'après la revendication 33, sachant que le système est agencé pour mettre à jour les informations de diagnostic incluses dans la représentation hiérarchique en utilisant les informations de diagnostic ajustées.

35. Le système d'après la revendication 25, sachant qu'un élément d'information de diagnostic sélectionnable à partir de la représentation hiérarchique est affiché avec un niveau d'alerte estimant une gravité en rapport avec la lésion candidate sélectionnée calculée sur la base de l'élément d'information de diagnostic.

36. Le système d'après la revendication 25, sachant que le résumé relatif à chaque catégorie d'information de diagnostic dans la représentation hiérarchique inclut un niveau d'alerte estimant une gravité en rapport avec la lésion candidate sélectionnée, calculée sur la base de la catégorie de l'information de diagnostic.

37. Le système d'après la revendication 25, sachant que le niveau d'alerte global estime un niveau de gravité général de la lésion candidate sélectionnée sur la base de toutes les catégories d'informations représentées dans la représentation hiérarchique.

38. Le système d'après la revendication 23, sachant que la quatrième région comprend en outre :
une première sous-zone configurée pour enregistrer un diagnostic concernant la lésion candidate sélectionné ; et
une deuxième sous-zone configurée pour enregistrer une confiance à l'égard du diagnostic.

39. Le système d'après la revendication 25, comprenant en outre des moyens pour activer l'intégration des informations de diagnostic sélectionnables dans la représentation hiérarchique pour aider à établir un diagnostic concernant une lésion sélectionnée.

40. Le système d'après la revendication 1, sachant que le mécanisme de manipulation de données visuelles est en outre capable de produire un rendu d'une page de rapport de maladie du foie qui comprend au moins un des éléments suivants :
une première portion configurée pour fournir des informations relatives au sujet ;
une deuxième portion configurée pour fournir des informations de diagnostic non visuelles associées à chaque lésion incluse dans une liste de lésions du foie;
une troisième portion configurée pour fournir des informations de diagnostic visuelles associées à chaque lésion incluse dans la liste des lésions du foie ; et
une quatrième portion configurée pour fournir un diagnostic pour chaque lésion incluse dans la liste des lésions du foie.

41. Le système d'après la revendication 40, sachant que la deuxième portion comprend en outre :
une première sous-portion configurée pour fournir chaque lésion incluse dans la liste des lésions du foie et les informations qui y sont associées ;
une deuxième sous-portion configurée pour fournir un résumé diagnostique ; et
une troisième sous-portion configurée pour fournir des preuves médicales à l'appui du diagnostic de la liste des lésions du foie.

42. Le système d'après la revendication 41, sachant que les informations associées à chaque lésion incluent au moins l'un des éléments suivants :
une estimation de l'emplacement de la lésion ;
une dimension estimée de la lésion ;
un volume estimé de la lésion ;
un diagnostic médical de la lésion ; et
une mesure indiquant une confiance dans le diagnostic médical.

43. Le système d'après la revendication 1, sachant que le mécanisme de manipulation des données comprend au moins un des éléments suivants :
un mécanisme de visualisation des données capable d'afficher un ensemble de données sur la base d'un paramètre d'affichage ;
un mécanisme d'amélioration des données pouvant être activé pour générer une version améliorée d'un ensemble de données ;
un mécanisme de détection de lésion pouvant être activé pour détecter une lésion candidate dans un ensemble de données ; et
un mécanisme d'extraction de caractéristiques pouvant être activé pour extraire une ou plusieurs caractéristiques associées à une lésion candidate détectée,
sachant que la version améliorée d'un ensemble de données est une image soustraite de l'intensité du foie (LIST) ou une image de tranche interpolée générée par interpolation basée sur plus d'une image de tranche correspondante identifiée à travers un ensemble de données à séquence temporelle.

44. Le système d'après la revendication 43, sachant que, dans le cas où la version améliorée d'un ensemble de données est une image LIST, la version améliorée d'un ensemble de données est obtenue en soustrayant des valeurs de pixels d'une première image LIST des valeurs de pixels correspondantes d'une deuxième image LIST pour chaque paire d'images LIST adjacentes.

45. Le système d'après la revendication 43, sachant que ladite détection d'une lésion candidate est effectuée dans l'un des modes suivants : un mode automatique, un mode interactif et un mode manuel.

46. Le système d'après la revendication 43, sachant que ladite extraction est effectuée dans l'un des modes suivants : un mode automatique, un mode interactif et un mode manuel.

47. Le système d'après la revendication 1, comprenant en outre un mécanisme de diagnostic de maladie du foie, qui comprend au moins l'un des éléments suivants :
un mécanisme de construction de représentation hiérarchique configuré pour générer une représentation hiérarchique d'informations de diagnostic visuelles et/ou non visuelles sélectionnables ;
un mécanisme interactif d'exploration des données capable de faciliter l'exploration des preuves diagnostiques en temps réel ; et
un mécanisme d'examen (*assessment*) de l'information capable de contribuer au rendu (*rendering*) des données en temps réel afin de faciliter l'examen de l'information.

48. Le système d'après la revendication 47, sachant qu'au moins une partie des informations de diagnostic dans la représentation hiérarchique est intégrée à un outil de manipulation des données.

49. Le système d'après la revendication 1, comprenant en outre un mécanisme de génération de rapport de diagnostic de maladie du foie capable d'être activé pour produire un rapport de diagnostic de maladie du foie.

50. Un procédé, comprenant le fait de :
charger un ou plusieurs ensembles de données visuelles sur le foie et des informations non visuelles associées à un sujet et spécifiques à une maladie du foie ;
activer une page de manipulation de données visuelles pour manipuler le ou les ensembles de données visuelles du foie, sachant que la page de manipulation de données visuelles comprend :
une première zone pour afficher des images d'un ou plusieurs ensembles de données et pour manipuler un ou plusieurs ensembles de données, et
une deuxième zone pour fournir une pluralité de moyens sélectionnables pour activer une ou plusieurs opérations de manipulation de données à effectuer en rapport avec le ou les ensembles de données affichés dans la première zone, sachant que
lorsque la première zone est configurée pour manipuler plus d'un ensemble de données, chaque image provient d'un ensemble de données correspondant et les images de différents ensembles de données peuvent être affichées de manière synchrone ;
visualiser dans la page de manipulation des données visuelles un ou plusieurs ensembles de données visuelles du foie récupérés, et
effectuer une ou plusieurs opérations de manipulation de données, activées via la pluralité de moyens sélectionnables affichés sur la page de manipulation de données visuelles, et devant être effectuées par rapport à un ou plusieurs ensembles de données, sachant que plus d'un ensemble de données peut être visualisé de manière synchronisée,
sachant qu'une vitesse d'affichage de différentes tranches d'un ensemble de données volumétriques dans la première zone est déterminée sur la base d'une distance entre une tranche affichée et une tranche candidate à la lésion.

51. Le procédé d'après la revendication 50, comprenant en outre le fait de sélectionner l'un des moyens sélectionnables pour effectuer une opération de manipulation de données correspondante, sachant que l'opération de manipulation de données sélectionnée comprend au moins l'un des éléments suivants :
avancer, dans une première direction, dans un ensemble de données volumétriques pour sélectionner une tranche à afficher dans la première zone ;
avancer, dans une deuxième direction, dans l'ensemble de données volumétriques pour sélectionner une tranche à afficher dans la première zone ;
ajuster un paramètre d'affichage associé à un ensemble de données affiché dans la première zone ; activer un processus de détection automatique de lésions appliqué à un ensemble de données affiché dans la première zone ;
activer un processus interactif de détection de lésions appliqué à un ensemble de données affiché dans la première zone ;
spécifier un ou plusieurs ensembles de données à afficher dans la première zone ;
manipuler des informations associées à une liste de lésions candidates détectées à partir d'un ensemble de données affiché dans la première zone ; et
supprimer des informations associées à une lésion candidate figurant sur la liste des candidats à la lésion.

52. Le procédé d'après la revendication 50, comprenant en outre l'activation d'une page de diagnostic de maladie du foie, qui permet au moins l'une des activités suivantes :
sélectionner une lésion candidate dans une liste de lésions candidates sélectionnables détectées à partir d'au moins un ensemble de données ;
afficher des informations visuelles relatives à la lésion candidate sélectionnée ;
explorer au moins un élément d'information de diagnostic associé à la lésion candidate sélectionnée et représenté par une hiérarchie d'informations de diagnostic sélectionnables ; et
établir un diagnostic concernant la lésion candidate sélectionnée sur la base d'au moins un élément d'information de diagnostic.

53. Le procédé d'après la revendication 52, sachant que l'information visuelle comprend un rendu 3D de la lésion candidate sélectionnée.

54. Le procédé d'après la revendication 52, sachant que la hiérarchie fusionne des informations de diagnostic visuelles et non visuelles.

55. Le procédé d'après la revendication 52, sachant qu'au moins une partie des informations de diagnostic dans la représentation hiérarchique est intégrée à un outil de manipulation de données.

56. Le procédé d'après la revendication 52, sachant que ledit établissement d'un diagnostic est effectué dans un mode automatique, un mode interactif et une combinaison de ceux-ci.

57. Le procédé d'après la revendication 56, sachant que ladite exploration comprend le fait de :
modifier un élément d'information de diagnostic pour produire un élément d'information de diagnostic mis à jour ; et
examiner un élément d'information de diagnostic différent relatif à l'élément d'information de diagnostic sur la base de l'élément d'information de diagnostic mis à jour;
évaluer un diagnostic pour la lésion sélectionnée, effectué sur la base de l'élément d'information de diagnostic en utilisant l'élément d'information de diagnostic mis à jour.

58. Le procédé d'après la revendication 57, sachant que ladite modification est réalisée dans l'un des modes suivants : mode manuel, mode interactif, mode automatique, et une combinaison de ceux-ci.

59. Le procédé d'après la revendication 57, sachant que ladite modification est réalisée en utilisant un outil de manipulation de données incorporé avec l'élément d'information de diagnostic.

60. Le procédé d'après la revendication 52, sachant que ladite exploration comprend le fait de :
détecter un ou plusieurs objets imagés dans un volume 3D sélectionné d'informations visuelles de diagnostic;
disséquer le volume 3D en une pluralité de parties, au moins une partie contenant au moins un des objets ; et
tirer, électroniquement, une des parties à l'écart des autres parties restantes pour visualiser une relation spatiale entre les objets.

61. Le procédé d'après la revendication 60, sachant que le ou les objets incluent au moins l'un des éléments suivants : un foie, une lésion et un vaisseau sanguin.

62. Le procédé d'après la revendication 60, comprenant en outre le fait de ré-assembler la partie détachée avec les autres parties restantes.

63. Le procédé d'après la revendication 60, comprenant en outre le fait de caractériser la relation spatiale.

64. Le procédé d'après la revendication 63, comprenant en outre le fait de dériver une décision médicale sur la base d'une caractérisation de la relation spatiale à partir de ladite caractérisation.

65. Le procédé d'après la revendication 64, sachant que la décision médicale inclut au moins l'un parmi un plan de traitement et un plan chirurgical en rapport avec la lésion sélectionnée.

66. Le procédé d'après la revendication 52, comprenant en outre le fait de :
afficher l'élément sélectionné d'information de diagnostic ;
présenter un résumé des informations de diagnostic associé à la lésion candidate sélectionnée ; et
afficher des informations relatives à un niveau d'alerte général concernant la lésion candidate sélectionnée.

67. Le procédé d'après la revendication 52, comprenant en outre le fait d'activer un moyen pour effectuer l'intégration d'un ou plusieurs éléments d'information de diagnostic sélectionnables à partir de la représentation hiérarchique pour aider à établir un diagnostic concernant une lésion sélectionnée.

68. Le procédé d'après la revendication 50, comprenant en outre le fait de générer un rapport de maladie du foie, qui comprend au moins un des éléments suivants :
des informations relatives au sujet ;
des informations de diagnostic non visuelles associées à chaque lésion incluse dans une liste de lésions du foie ;
des informations de diagnostic visuelles associées à chaque lésion incluse dans la liste des lésions du foie ; et
un diagnostic pour chaque lésion figurant dans la liste des lésions du foie.
